(19) Europäisches Patentamt
European Patent Office
Office européen des brevets

(11) **EP 4 410 206 A1**

## (12) EUROPEAN PATENT APPLICATION
published in accordance with Art. 153(4) EPC

(43) Date of publication:
**07.08.2024 Bulletin 2024/32**

(21) Application number: **22876134.2**

(22) Date of filing: **26.09.2022**

(51) International Patent Classification (IPC):
**A61B 5/257** (2021.01)   **A61B 5/263** (2021.01)
**H01B 1/20** (2006.01)

(52) Cooperative Patent Classification (CPC):
**A61B 5/257; A61B 5/263; H01B 1/20**

(86) International application number:
**PCT/JP2022/035748**

(87) International publication number:
**WO 2023/054267 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 JP 2021159960**

(71) Applicant: **Nitto Denko Corporation
Ibaraki-shi, Osaka 567-8680 (JP)**

(72) Inventors:
• **MINAKATA, Masayuki
Ibaraki-shi, Osaka 567-8680 (JP)**
• **YANO, Chiharu
Ibaraki-shi, Osaka 567-8680 (JP)**

(74) Representative: **Hoffmann Eitle
Patent- und Rechtsanwälte PartmbB
Arabellastraße 30
81925 München (DE)**

(54) **ELECTRODE PAD AND BIOMETRIC SENSOR**

(57)   An electrode pad includes a first conductive layer, and a second conductive layer provided on one surface of the first conductive layer, wherein the first conductive layer has an elongation at break of 50% to 600%, and wherein the second conductive layer contains 0.1% by mass to 40% by mass of a metal chloride.

FIG.1

EP 4 410 206 A1

**Description**

TECHNICAL FIELD

The present invention relates to an electrode pad and a living body sensor.

BACKGROUND OF THE INVENTION

In order to acquire an electric signal regarding biometric information of a subject by bringing electrodes used for a living body sensor or the like into contact with the skin of the subject, a method of suppressing impedance between the electrodes and the skin to be low by improving adhesiveness to the skin and followability to deformation of the skin caused by body motion such as walking or ascending and descending of a staircase, rubbing of clothes, or the like has been studied.

As a method of suppressing the impedance between the skin and the electrodes, for example, a living body sensor is disclosed which includes a pressure-sensitive adhesive layer having an attachment surface to be attached to a subject and electrodes formed of a conductive polymer and exposed from holes in the attachment surface, and acquires biometric information with the electrodes exposed from the holes in the attachment surface while the pressure-sensitive adhesive layer is attached to the skin (for example, refer to Patent Document 1).

RELATED-ART DOCUMENT

Patent Documents

[Patent Document 1] Japanese Patent Application Laid-Open No. 2020-156692

SUMMARY OF INVENTION

Problem to be solved by the invention

However, in the living body sensor of Patent Document 1, if a gap is formed between the hole provided in the attachment surface of the pressure-sensitive adhesive layer and the electrode, a gap is formed between the electrode and the attachment surface, and the skin, and the impedance between the electrode and the skin may be increased.

Therefore, in order to stably acquire an electrical signal regarding a biometric information for a long period of time, it is important to maintain the impedance between the electrodes and the living body surface at a low level, because living body sensors are often attached to the skin or other biological surfaces and used for long periods of time. Therefore, when the electrode is used in the living body sensor, a method capable of maintaining a low impedance between the electrode and the surface of the living body has been desired.

An aspect of the present invention is to provide an electrode pad that can maintain a low impedance between an electrode and a living body surface when used in a living body sensor.

Means for Solving Problem

In an aspect of the electrode pad according to the present invention, the electrode pad includes a first conductive layer, and a second conductive layer provided on one surface of the first conductive layer, wherein the first conductive layer has an elongation at break of 50% to 600%, and wherein the second conductive layer contains 0.1% by mass to 40% by mass of a metal chloride.

Effects of the Invention

According to the aspect of the invention, when the electrode pad is used in a living body sensor, the low impedance between the electrode and the surface of the living body can be maintained.

BRIEF DESCRIPTION OF THE DRAWINGS

[FIG. 1] FIG. 1 is a perspective view illustrating a structure of an electrode pad according to an embodiment of the present invention;

[FIG. 2] FIG. 2 is a view taken in the direction of I-I in FIG. 1;

[FIG. 3] FIG. 3 is a view taken in the direction of II-II in FIG. 1;

[FIG. 4] FIG. 4 is a conceptual view illustrating a change in viscosity of a fluid with respect to a magnitude of a force to be applied for each type of fluid;

[FIG. 5] FIG. 5 is an explanatory view illustrating an example of an electrocardiographic waveform without noise;

[FIG. 6] FIG. 6 is a perspective view illustrating the structure of a living body sensor including the electrode pad according to an embodiment of the present invention;

[FIG. 7] FIG. 7 is a plan view schematically illustrating the structure of the living body sensor; [FIG. 8] FIG. 8 is a cross-sectional view taken along line I-I of FIG. 6; and

[FIG. 9] FIG. 9 is an explanatory view illustrating a state in which the living body sensor is attached to skin of a living body.

## DETAILED DESCRIPTION OF THE INVENTION

Hereinafter, embodiments of the present invention will be described in detail. In order to facilitate understanding of the description, the same components are denoted by the same reference numerals in the drawings, and redundant description will be omitted. In addition, the scale of each member in the drawings may be different from the actual scale. In the present specification, "to" indicating a numerical range means that numerical values described before and after "to" are included as a lower limit value and an upper limit value, unless otherwise specified.

<Electrode Pad>

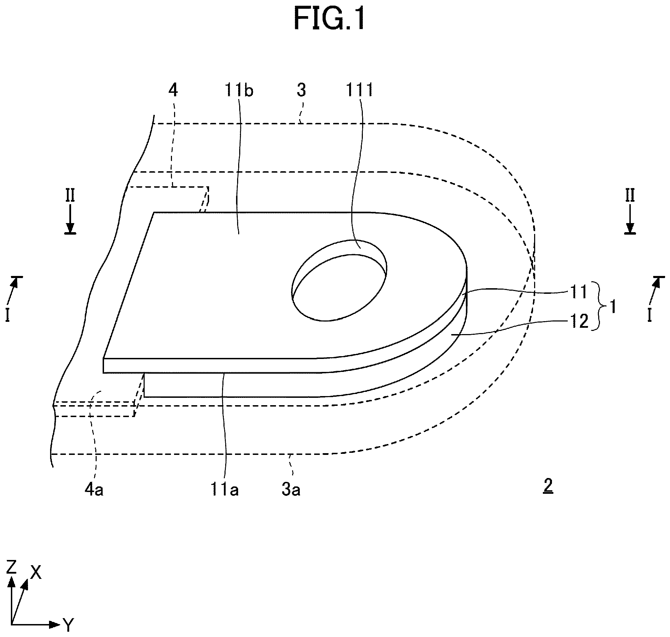

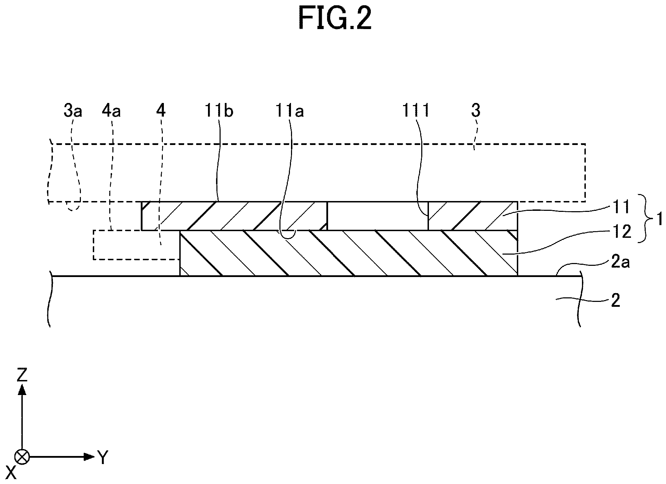

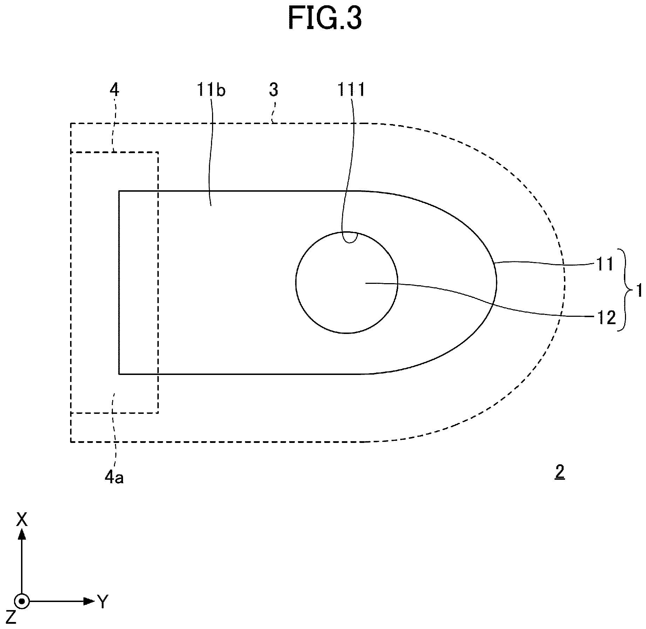

The electrode pad according to the present embodiment will be described. FIG. 1 is a perspective view illustrating a configuration of the electrode pad according to the present embodiment, FIG. 2 is a cross-sectional view of the electrode pad as viewed in the direction I-I of FIG. 1, and FIG. 3 is a plan view of the electrode pad as viewed in the direction II-II of FIG. 1. As illustrated in FIGS. 1 and 2, an electrode pad 1 includes an electrode 11 as a first conductive layer and a second conductive layer 12 provided on a part of one (lower) main surface (lower surface) 11a of the electrode 11. The second conductor layer 12 of the electrode pad 1 is attached to skin 2a, which is a surface (adherend) of a subject (person) 2, and a potential difference (polarization voltage) between the skin 2a and the electrode 11 is measured to measure an electric signal related to biometric information of the subject 2.

In the present specification, a three-dimensional orthogonal coordinate system in three axial directions (X-axis direction, Y-axis direction, and Z-axis direction) is used. The short-axis direction of the electrode 11 is the X-axis direction, the long-axis direction is the Y-axis direction, and the height direction (thickness-wise direction) is the Z-axis direction. The direction opposite to the side (attachment side) where the electrode pad 1 is to be attached to the subject 2 is defined as the +Z-axis direction, and the attachment side of the electrode pad 1 is defined as the -Z-axis direction. In the following description, for convenience of description, the +Z-axis direction side may be referred to as an upper side or an upper portion, and the -Z-axis direction side may be referred to as a lower side or a lower portion, but these do not represent a universal vertical relationship.

The living body refers to a human body (person), an animal such as a cow, a horse, a pig, a chicken, a dog, or a cat, or the like. The electrode pad according to the present embodiment can be suitably used for a living body, particularly for a human body. In the present embodiment, a case where the living body is a human will be described as an example.

The inventor of the present application has focused on the fact that, when the electrode 11 is applied to a living body sensor, if a gap is formed between the electrode 11 and the skin 2a, the impedance between the electrodes 11 and the skin 2a increases, which affects the measurement of biometric information. The present inventors have found that, by disposing the second conductive layer 12 having conductivity between the electrode 11 and the skin 2a, a gap between the electrode 11 and the skin 2a is not appreciably generated due to the presence of the second conductive layer 12, and a polarization voltage generated by a difference in charge transfer between the skin 2a and the electrode 11 to reduce a potential difference therebetween is reduced, thereby easily conducting between the electrode 11 and the skin 2a. On the other hand, the electrode 11 needs to have appropriate flexibility and be able to adhere to the second conductive layer 12. The inventors of the present application have developed the electrode pad 1 in which the second conductive layer 12 is provided on the lower surface 11a of the electrode 11, which is on the surface on the living body side. The inventors have also found that the low impedance between the electrode 11 and the skin 2a can be maintained while maintaining adhesion between the electrode 11 and the second conductive layer 12, and biological information can be transmitted from the skin 2a to the electrode 11 via the second conductor layer 12, enabling stable measurement of electrical signals related to biological information, when the electrode 11 has an elongation at break of 50% to 600% and the second conductive layer 12 contains 0.1% by mass to 40% by mass of a metal chloride.

The electrode pad 1 may have any shape such as a sheet shape or the like.

As illustrated in FIGS. 1 and 2, the other (upper) main (top) surface 11b of the electrode 11 may be attached to a main (bottom) surface 3a of an adhesive layer 3.

The electrode 11 may be provided such that the end of the electrode 11 on the -Y-axis direction side is laminated on the upper side of one main surface (upper surface) 4a of a wiring 4. The electrode pad 1 transmits an electrical signal related to biometric information acquired from the subject 2 to the outside through the wiring 4.

The impedance (contact impedance) of the electrode pad 1 when the skin 2a of the subject 2 is in contact with the electrode pad 1 is preferably 2.0 M$\Omega$ or less, more preferably 1.0 M$\Omega$ or less, even more preferably 0.5 M$\Omega$ or less, and most preferably 0.2 M$\Omega$ or less. As the contact impedance is lower, an electric signal relating to biometric information can be detected with less noise. When an electrocardiograph is acquired as biometric information, if the contact impedance is 2.0 M$\Omega$ or less, the electrocardiograph can be detected with less noise in the obtained electrocardiographic waveform.

The polarization voltage of the electrode pad 1 is preferably 0.1 mV to 100 mV, more preferably 10 mV to 95 mV, and even more preferably 40 mV to 90 mV. When the polarization voltage is within the above-described preferable range, an electric signal relating to the biometric information can be acquired, and the contact impedance can be maintained at 1 M$\Omega$ or less, and thus the polarization voltage can be suitably used when an electrocardiograph is acquired as the biometric information.

A method of measuring the polarization voltage of the electrode pad 1 is not particularly limited, and any appropriate method can be used. For example, the electrode 11 immersed in a mixed solution containing a metal chloride (for example, NaCl) in distilled water is assumed as the electrode pad 1 in which the second conductive layer 12 is laminated on the electrode 11. The polarization voltage of the electrode pad 1 is measured by immersing a silver chloride electrode as a reference electrode in addition to the electrode 11 in this mixed solution and measuring the potential difference between the electrode 11 and the silver chloride electrode. The measured potential difference can be assumed as the polarization voltage of the electrode pad 1.

(Electrode)

As illustrated in FIG. 2, the electrode 11 may be attached at its upper surface 11b to a part of one lower surface 3a of the adhesive layer 3, and one end portion (in the -Y-axis direction) of the lower surface 11a and the vicinity thereof may be laminated on the wiring 4. The electrode 11 may have any shape such as a sheet shape or the like.

The shape of the electrode 11 in a plan view is not particularly limited, and may be designed to be any shape as appropriate according to the application or the like. As illustrated in FIG. 3, the electrode 11 may be formed in an arc shape on one end side (+Y-axis direction) and in a rectangular shape on the other end side (-Y-axis direction) in a plan view.

As illustrated in FIG. 3, the electrode 11 may have a circular through hole 111 in the lower surface 11a. Thus, the electrode 11 can expose the adhesive layer 3 from the through hole 111 to the attachment side in a state where the electrode 11 is attached to the adhesive layer 3, and thus the adhesion between the electrode 11 and the skin 2a can be improved. The number of the through holes 111 may be plural.

The electrode 11 has an elongation at break of 50% to 600%. If the elongation at break of the electrode 11 is less than 50%, the electrode 11 becomes too hard. Therefore, vibration or deformation of the electrode pad 1 during use is caused, thereby the second conductive layer 12 is deformed due to deformation of the skin 2a, the electrode 11 cannot be sufficiently deformed following the deformation of the second conductive layer 12, a gap is likely to be formed between the through hole 111 in the electrode 11 and the adhesive layer 3, and the impedance between the electrode 11 and the skin 2a is likely to increase. When the elongation at break of the electrode 11 exceeds 600%, the electrode 11 becomes too soft, thus the electrode 11 is easily peeled off from the adhesive layer 3 or the wiring 4. The elongation at break is preferably from 100% to 500%, more preferably from 200% to 450%, and even more preferably from 250% to 400%.

The elongation at break represents the degree of expansion and contraction of the electrode 11 when the electrode 11 is pulled. The elongation at break is also referred to as a tensile elongation at break. The tensile elongation at break of the electrode 11 is expressed as the difference ($L-L_0$) between the length $L$ of the electrode 11 in the long axial direction or short axial direction at a breakage and the length $L_0$ of the electrode 11 in the long axial direction or short axial direction before the electrode 11 is pulled, divided by the length $L_0$ of the electrode 11 in the long axial direction or short axial direction before the electrode 11 is pulled, as in the following formula (1) expressed as a percentage.

$$\text{Elongation at break (\%)} = (L-L_0)/L_0 \times 100 \quad \ldots \quad (1)$$

The elongation at break of the electrode 11 can be determined in accordance with, for example, JIS C 2151, ASTM D 882, or the like. The elongation at break of the electrode 11 can be measured by pulling both ends of the electrode 11 using a tensile tester (for example, AGS-J, manufactured by Shimadzu Corporation) and measuring the elongation at the time when the electrode 11 is cut or broken. The tensile test condition can be set appropriately. For example, the tensile test can be performed with the electrode 11 having a width (the maximum width of the electrode 11 in the short axial direction) of 10 mm and the tensile strength of 300 mm/min.

The elongation at break of the electrode 11 may be an average value of the measured values of a plurality of (for example, three) electrodes 11.

In the present embodiment, the electrode 11 has a circular outer shape on one side and a rectangular outer shape on the other side, and when the electrode 11 is attached to the skin 2a, the electrode 11 is most likely to expand and contract in the long axial direction. Therefore, the elongation at break of the electrode 11 is preferably the elongation at break in the long axial direction of the electrode 11. When the outer shape of the electrode 11 is rectangular, the elongation at break of the electrode 11 is preferably the elongation at break in the long-side direction of the electrode 11. When the outer shape of the electrode 11 is a square, the elongation at break of the electrode 11 may be the elongation at break in one side direction of the electrode 11. When the outer shape of the electrode 11 is circular, the elongation at break of the electrode 11 may be the elongation at break of the diameter of the electrode 11.

The electrode 11 can be formed using a cured product of a conductive composition containing a conductive polymer and a binder resin, a metal, an alloy, or the like. Among these, the electrode 11 is preferably formed using a cured product of the conductive composition from the viewpoint of safety of a living body, such as preventing an allergic reaction or the like from occurring when the electrode pad 1 is applied to a living body.

The cured product of the conductive composition contains a conductive polymer and a binder resin, and may contain the conductive polymer in a state of being dispersed in the binder resin.

As the conductive polymer, for example, a polythiophene-based conductive polymer, a polyaniline-based conductive polymer, a polyacetylene-based conductive polymer, a polypyrrole-based conductive polymer, a polyphenylene-based conductive polymer, derivatives thereof, composites thereof, and the like can be used. These may be used alone or in combination of two or more. Among these, a composite in which polythiophene is doped with polyaniline as a dopant is preferable. Among the complexes of polythiophene and polyaniline, PEDOT/PSS obtained by doping poly(3,4-ethylenedioxythiophene) (also referred to as PEDOT) as polythiophene with polystyrene sulfonic acid (poly 4-styrene sulfonate; PSS) as polyaniline is more preferably used from the viewpoint of low contact impedance with a living body and high conductivity.

A content of the conductive polymer is preferably 0.20 parts by mass to 20 parts by mass, more preferably 2.5 parts by mass to 15 parts by mass, and even more preferably 3.0 parts by mass to 12 parts by mass, with respect to 100 parts by mass of the conductive composition. When the content is within the above-described preferable range with respect to the conductive composition, the conductive composition can have excellent conductivity, toughness, and flexibility.

The conductive polymer may be a solid formed into a pellet shape. In this case, a part of the solid of the conductive polymer may be dissolved in a solvent within a range in which the content of each component in the conductive composition does not vary greatly.

The conductive polymer may be used as an aqueous solution obtained by dissolving the conductive polymer in a solvent. In this case, either an organic solvent or an aqueous solvent can be used as the solvent. Examples of the organic solvent include ketones such as acetone and methyl ethyl ketone (MEK); esters such as ethyl acetate; ethers such as propylene glycol monomethyl ether; and amides such as N,N-dimethylformamide. Examples of the aqueous solvent include water; and alcohols such as methanol, ethanol, propanol, and isopropanol. Among them, the aqueous solvents are preferable.

As the binder resin, a water-soluble polymer or a water-insoluble polymer can be used. As the binder resin, a water-soluble polymer is preferably used from the viewpoint of compatibility with other ingredients contained in the conductive composition. The water-soluble polymer may be a polymer that is not completely dissolved in water and has hydrophilicity (hydrophilic polymer).

As the water-soluble polymer, a hydroxyl group-containing polymer or the like can be used. Examples of the hydroxyl group-containing polymer include saccharides such as agarose, polyvinyl alcohol (PVA), modified polyvinyl alcohol, polypyrrole, and a copolymer of acrylic acid and sodium acrylate. These may be used alone or in combination of two or more thereof. Among them, polyvinyl alcohol or modified polyvinyl alcohol is preferable, and modified polyvinyl alcohol is more preferable.

The content of the binder resin is preferably 5 parts by mass to 140 parts by mass, more preferably 10 parts by mass to 100 parts by mass, and even more preferably 20 parts by mass to 70 parts by mass with respect to 100 parts by mass of the conductive composition. When the content is within the above preferable range with respect to the conductive composition, a cured product obtained using the conductive composition can have excellent conductivity, toughness, and flexibility.

The binder resin may be used as an aqueous solution in which the binder resin is dissolved in a solvent. As the solvent, the same solvent as that in the case of the conductive polymer described above may be used.

The conductive composition preferably further contains at least one of a crosslinking agent or a plasticizer.

Each of the crosslinking agent and the plasticizer has a function of imparting toughness and flexibility to a cured product obtained by using the conductive composition.

The toughness is a property including both excellent strength and an excellent degree of elongation. The toughness should not be a property in which one of strength and a degree of elongation is remarkably excellent but the other is remarkably low, and may be a property in which a balance between strength and a degree of elongation is excellent.

The flexibility is a property of suppressing occurrence of damage such as breakage at a bent portion after a cured product containing the conductive composition is bent.

The crosslinking agent has a function of crosslinking the binder resin. When the crosslinking agent is contained in the binder resin, the toughness of a cured product obtained by using the conductive composition can be improved. The crosslinking agent is preferably reactive with a hydroxyl group. If the crosslinking agent has reactivity with a hydroxyl group, when the binder resin is a hydroxyl group-containing polymer, the crosslinking agent can react with the hydroxyl group of the hydroxyl group-containing polymer.

Examples of the crosslinking agent include zirconium compounds such as zirconium salts; titanium compounds such as titanium salts; boron compounds such as a boric acid; isocyanate compounds such as a blocked isocyanate; aldehyde compounds such as sodium glyoxylate, formaldehyde, acetaldehyde, glyoxal, and glutaraldehyde; alkoxyl group-containing compounds; and methylol group-containing compounds. These may be used alone or in combination of two or more thereof. Among them, when the binder resin is polyvinyl alcohol, sodium glyoxylate is preferable from the viewpoint of easily forming a crosslinked structure by reacting with the polyvinyl alcohol and easily maintaining the performance of the cured product obtained by using the conductive composition.

Since the crosslinking agent is an optional ingredient, it is not necessarily contained in the conductive composition, and the content of the crosslinking agent may be 0 parts by mass. When the conductive composition contains the crosslinking agent, the content of the crosslinking agent is preferably 0.01 parts by mass to 1.5 parts by mass, more preferably 0.2 parts by mass to 1.2 parts by mass, and even more preferably 0.4 parts by mass to 1.0 parts by mass with respect to 100 parts by mass of the conductive composition. When the content is within the preferable range described above, a cured product obtained using the conductive composition can have excellent toughness and flexibility.

The crosslinking agent may be used as an aqueous solution obtained by dissolving the crosslinking agent in a solvent. As the solvent, the same solvent as that in the case of the conductive polymer described above can be used.

The plasticizer has a function of improving the conductivity of a cured product obtained by using the conductive composition, and also, improving the degree of tensile elongation and flexibility. Examples of the plasticizer include polyol compounds such as glycerin, ethylene glycol, propylene glycol, sorbitol, and polymers thereof; and aprotic compounds such as N-methylpyrrolidone (NMP), dimethyl formaldehyde (DMF), N-N'-dimethylacetamide (DMAc), and dimethyl sulfoxide (DMSO). These may be used alone or in combination of two or more kinds thereof. Among them, glycerin is preferable from the viewpoint of compatibility with another ingredient.

The content of the plasticizer is preferably 0.2 parts by mass to 150 parts by mass, more preferably 1.0 parts by mass to 90 parts by mass, and even more preferably 10 parts by mass to 70 parts by mass with respect to 100 parts by mass of the conductive composition. When the content is within the preferable range described above, a cured product obtained using the conductive composition can have excellent toughness and flexibility.

When the conductive composition contains at least one of the crosslinking agent or the plasticizer, a cured product obtained using the conductive composition can be improved in toughness and flexibility.

When the conductive composition contains the crosslinking agent but does not contain the plasticizer, a cured product obtained by using the conductive composition is such that toughness can be improved, that is, both tensile strength and the degree of tensile elongation can be improved, and also flexibility can be improved.

When the conductive composition contains the plasticizer but does not contain the crosslinking agent, the degree of tensile elongation of a cured product obtained by using the conductive composition can be improved, and thus, as a whole, toughness of the cured product obtained by using the conductive composition can be improved. In addition, flexibility of the cured product obtained by using the conductive composition can be improved.

The conductive composition preferably contains both the crosslinking agent and the plasticizer. When the conductive composition contains both the crosslinking agent and the plasticizer, a cured product obtained using the conductive composition can have even more excellent toughness.

The conductive composition may contain, in addition to the above-described ingredients, any one or ones of various known additives such as a surfactant, a softening agent, a stabilizer, a leveling agent, an antioxidant, a hydrolysis inhibitor, a swelling agent, a thickener, a colorant, and a filler at an appropriate content rate as needed. Examples of the surfactant include silicone-based surfactants.

The conductive composition is prepared by mixing the above-described ingredients at the above-described ratio.

The conductive composition can contain a solvent at an appropriate content rate as necessary. Thereby, an aqueous solution of the conductive composition (conductive composition aqueous solution) is prepared.

As the solvent, an organic solvent or an aqueous solvent can be used. Examples of the organic solvent include ketones such as acetone and methyl ethyl ketone (MEK); esters such as ethyl acetate; ethers such as propylene glycol monomethyl ether; and amides such as N,N-dimethylformamide. Examples of the aqueous solvent include water; and alcohols such as methanol, ethanol, propanol, and isopropanol. Among them, the aqueous solvents are preferable.

(Second Conductive Layer)

The second conductive layer 12 is provided on a part of the lower surface 11a of the electrode 11. The second conductive layer 12 is a water-containing substance having conductivity and fluidity, and may be in a gel form.

The second conductive layer 12 includes a metal chloride. As the metal contained in the metal chloride, Cu, Na, Sn, Al, Si, K, Ag, Ca, or the like can be used. These may be used alone or in combination of two or more. Among these, Na, K, and Ca are preferable in terms of the potential difference from PEDOT/PSS when the electrode 11 contains PEDOT/PSS as the conductive polymer, ease of handling, and manufacturing cost.

Examples of the metal chloride include copper chloride ($CuCl_2$), sodium chloride (NaCl), tin chloride ($SnCl_2$), aluminum chloride ($AlCl_3$), silicone trichloride ($SiHCl_3$), potassium chloride (KCl), silver chloride (AgCl), calcium chloride ($CaCl_2$), and the like.

The content of the metal chloride is 0.1% by mass to 40.0% by mass, preferably 0.5% by mass to 30.0% by mass, more preferably 3.0% by mass to 25.0% by mass, and even more preferably 5.0% by mass to 20.0% by mass. When the content of the metal chloride is within the above-described preferable range, the transfer of charges between the second conductive layer 12 and the skin 2a of the subject 2 can be assisted, and the potential difference can be suppressed.

The metal chloride can be identified by observing a peak derived from the metal chloride using gas chromatography mass spectrometry (GC/MS).

The second conductive layer 12 may include a viscous fluid. The viscous fluid includes, for example, a non-Newtonian fluid having a viscous property in which the relationship between the shear stress of the flow and the flow velocity gradient is not linear (the shear stress of the flow is not proportional to the shear velocity of the flow).

Non-Newtonian fluids include Bingham fluids and pseudoplastic fluids. A fluid having a viscous property in which the shear stress of a flow is proportional to the shear rate of the flow is a Newtonian fluid.

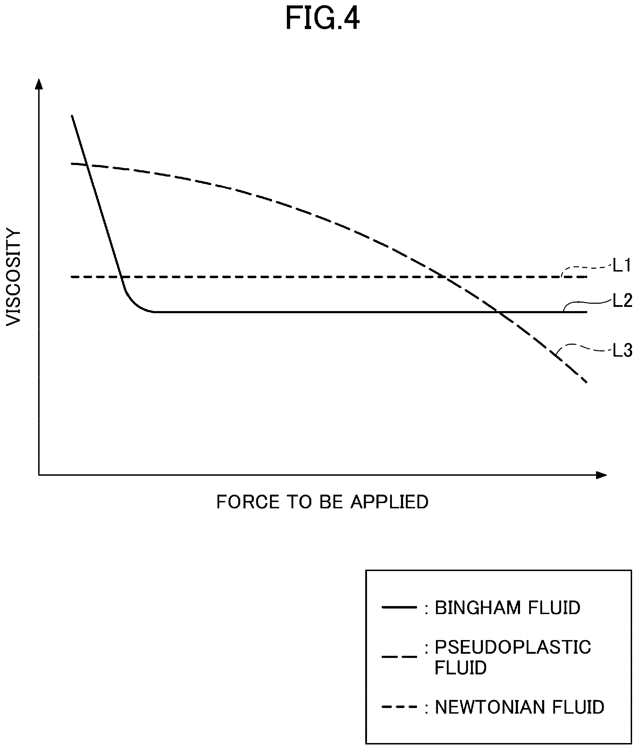

FIG. 4 is a conceptual view illustrating an example of a change in the viscosity of the viscous fluid with respect to the magnitude of the force applied to the viscous fluid. In FIG. 4, a line L1 indicates a Newtonian fluid, a line L2 indicates a Bingham fluid, and a line L3 indicates a pseudoplastic fluid.

As illustrated in FIG. 4, in the Newtonian fluid, the viscosity of the fluid is constant regardless of the magnitude of the applied force (see line L1). That is, when the viscous fluid has the property of a Newtonian fluid, the viscosity of the viscous fluid does not change even when a force is applied to the second conductive layer 12 in the electrode pad 1 illustrated in FIG. 1.

In the Bingham fluid, the viscosity of the fluid decreases as the magnitude of the applied force increases until the magnitude of the applied force reaches a predetermined value, and after the magnitude of the applied force reaches the predetermined value, the viscosity of the fluid is constant without depending on the magnitude of the applied force, and when the surface of the cured material containing viscous fluid is pressed, the cured material is softened. That is, when the viscous fluid has the property of a Bingham fluid, in the electrode pad 1, the second conductive layer 12 can easily flow in the state where the shear stress reaches a predetermined value, and therefore the second conductive layer 12 can be easily formed on the lower surface 11a of the electrode 11. On the other hand, in a state where the shear stress does not reach the predetermined value, the flow of the second conductive layer 12 tends to be suppressed, and thus the second conductive layer 12 can maintain the curved state and the flat state.

In the pseudoplastic fluid, the viscosity of the viscous fluid decreases as the magnitude of the applied force increases, and the pseudoplastic fluid has a property of softening when the surface of the cured product containing the viscous fluid is pressed. That is, if the viscous fluid has the properties of a pseudoplastic fluid (pseudoplastic viscosity), in the electrode pad 1, the second conductive layer 12 is more difficult to move without force and easier to move with force. For example, when the second conductive layer 12 contains a pseudoplastic fluid, the second conductive layer 12 is easy to apply on the lower surface 11a of the electrode 11 to form a predetermined thickness in a predetermined range, and after forming on the lower surface 11a of the electrode 11, the second conductive layer 12 can be kept adhered to the skin 2a.

Therefore, when the second conductive layer 12 contains a pseudoplastic fluid or a Bingham fluid as the viscous fluid, the viscosity of the viscous fluid is preferably constant or reduced after the second conductive layer 12 is formed on the lower surface 11a of the electrode 11.

The material of the viscous fluid is not particularly limited as long as the second conductive layer 12 can exhibit the above-described function. Specifically, one or more of water, propylene glycol, mineral oil, glyceryl monostearate, polyoxyethylene stearate, stearyl alcohol, methylparaben, butylparaben, propylparaben, and the like may be used as the viscous fluid.

When the second conductive layer 12 is a pseudoplastic fluid or a Bingham fluid, the viscosity of the second conductive layer 12 is preferably 1 mPas to 100,000 mPas, more preferably 300 mPas to 20,000 mPas, and even more preferably 700 mPas to 5,000 mPas.

The height of the second conductive layer 12 can be set to any height as appropriate, but is preferably a height that is substantially equal to the lower surface of a member (for example, an adhesive sheet) or the like provided on the lower surface of the wiring 4, for example, from the viewpoint of maintaining flexibility while ensuring conductivity between the electrode 11 and the skin 2a.

An example of a method of manufacturing the electrode pad 1 will be described. Note that here, a cured product obtained using a conductive composition is used for the electrode 11.

First, the electrode 11 formed into a predetermined shape is prepared.

The conductive polymer and the binder resin are mixed at the above-mentioned ratio to prepare a conductive composition containing the conductive polymer and the binder resin. The conductive composition may further contain at least one of the crosslinking agent or the plasticizer at the above-described rate. When the conductive composition is prepared, the conductive polymer, the binder resin, and the crosslinking agent may be used as an aqueous solution obtained from being dissolved in a solvent.

The conductive composition may contain, if necessary, a solvent at an appropriate rate in addition to the solvent containing the conductive polymer, the binder resin, and the crosslinking agent, and thus, an aqueous solution of the conductive composition (a conductive composition aqueous solution) may be used. As the solvent, the same solvent as that described above can be used.

After the conductive composition is applied to a surface of a release base, the conductive composition is heated to cause a crosslinking reaction of the binder resin contained in the conductive composition to proceed, resulting in the binder resin being cured. As a result, a cured product of the conductive composition is obtained. If necessary, a surface of the obtained cured product is punched (pressed) using a press machine or the like to form one or more through-holes through the surface of the cured product and form the outer shape of the cured product into a predetermined shape. As a result, the electrode 11, each of which is a shaped body having one or more through-holes through the surface thereof and having a predetermined outer shape is obtained. Instead of the press machine, a laser processing machine may be used for the shaping. In this regard, only the one or more through-holes may be formed through the surface of the obtained cured product, or only the outer shape may be made into a predetermined shape. In a case where the cured product can be used as the electrode 11 as it is, the cured product may be used as the electrode 11 without being subjected to a shaping process or the like.

Note that the conductive polymer, the binder resin, the crosslinking agent, and the plasticizer contained in the electrode 11 have contents equivalent to those having been added at the time of the preparation of the conductive composition.

As the release base, a release liner, a core material, or the like can be used. As the release liner, a resin film such as a polyethylene terephthalate (PET) film, a polyethylene (PE) film, a polypropylene (PP) film, a polyamide (PA) film, a polyimide (PI) film, or a fluororesin film can be used. As the core material, a resin film such as a PET film or a PI film; a ceramic sheet; a metal film such as an aluminum foil; a resin substrate reinforced with a glass fiber or a plastic non-woven fiber; a silicone substrate or a glass substrate can be used.

Examples of a method of applying the conductive composition onto the release base include roll coating, screen coating, gravure coating, spin coating, reverse coating, bar coating, blade coating, air knife coating, dipping, dispensing, and the like, and a method in which a small amount of the conductive composition is dropped onto the base and then spread with a doctor blade. By any of these coating methods, the conductive composition is uniformly coated on the release base.

As a method of heating the conductive composition, a known dryer such as a drying oven, a vacuum oven, an air circulation type oven, a hot air dryer, a far infrared dryer, a microwave reduced pressure dryer, or a high frequency dryer can be used.

The heating condition should be a condition under which the crosslinking agent contained in the conductive composition can react.

The heating temperature with respect to the conductive composition is a temperature at which curing of the binder resin contained in the conductive composition can proceed. The heating temperature is preferably 100°C to 200°C. In the case where the conductive composition contains the crosslinking agent, when the heating temperature is in the range of 100°C to 200°C, reaction of the crosslinking agent easily proceeds and curing of the binder resin can be accelerated.

The heating time of the conductive composition is preferably 0.5 minutes to 300 minutes, and more preferably 5 minutes to 120 minutes. When the heating time is in the range of 0.5 minutes to 300 minutes, the binder resins can be disposed on the lower surface 11a of the electrode 11.

Next, the second conductive layer 12 is formed by applying a second conductive layer forming composition containing a mixture of a metal chloride and a viscous fluid to at least a part of the lower surface 11a of the prepared electrode 11. Thus, the electrode pad 1 illustrated in FIG. 1 is obtained.

The method of applying the second conductive layer forming composition can be performed under the same conditions as in the method of applying the conductive composition described above.

The electrode pad 1 according to the present embodiment includes an electrode 11 and a second conductive layer 12. The electrode 11 has an elongation at break of 50% to 600% and thus has high stretchability. Even when the second conductive layer 12 is deformed by deformation of the skin 2a caused by body motion of the subject 2, the electrode 11 is flexibly deformed by the deformation of the second conductive layer 12 and can maintain the bonded state. Therefore, when the electrode pad 1 is used by being attached to the skin 2a, even if the skin 2a moves due to the body motion of the living body, the electrode pad 1 is deformed following the movement of the skin 2a, and the contact state with the skin 2a can be maintained. The second conductive layer 12 contains 0.1% by mass to 40% by mass of the metal chloride, and thus has conductivity and can secure conductivity between the skin 2a and the electrode 11. In addition, the second conductive layer 12 can reduce a polarization voltage generated by a difference in charge transfer between the skin 2a and the electrode 11 and assist the charge transfer between the skin 2a and the electrode 11. Therefore, the second conductive layer 12 can reduce a potential difference between the skin 2a and the electrode 11 and easily conduct between the skin 2a and the electrode 11. Furthermore, since the second conductive layer 12 is a hydrated material having high fluidity, the second conductive layer 12 can be deformed to sufficiently follow small irregularities of the skin 2a, and thus it is possible to reduce the occurrence of a gap between the second conductive layer 12 and the skin 2a. Even if the skin 2a moves due to the body motion of the subject 2 when the electrode pad 1 is attached to the skin 2a of the subject 2, the second conductive layer 12 is deformed following the movement of the skin 2a, and the contact state with the skin 2a can be maintained while reducing the generation of a gap between the second conductive layer 12 and the skin 2a. Therefore, when the electrode pad 1 is used for a living body sensor, the impedance between the electrode 11 and the skin 2a can be prevented from increasing and varying, and thus the impedance between the electrode 11 and the skin 2a can be maintained at a low level.

Therefore, when the electrode pad 1 is used as a living body sensor to measure electrocardiographs, it is possible to suppress an increase and variation in noise generated during measurement, and thus it is possible to maintain a high average value of RR intervals of electrocardiographs, for example, 0.5 or more.

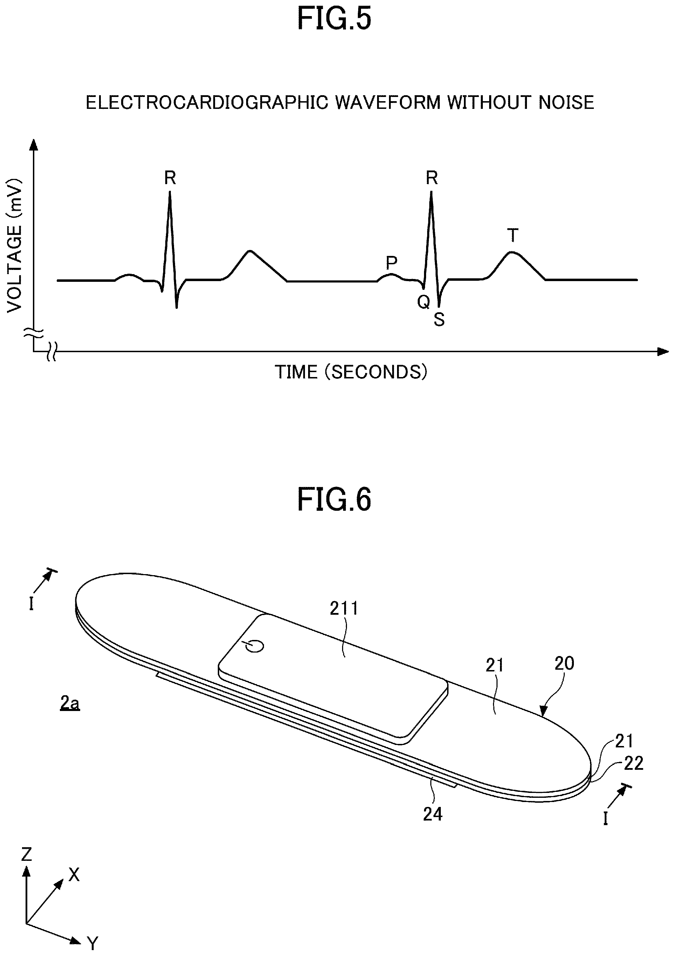

The average value of the RR intervals is an average value of intervals between adjacent R waves of an electrocardiograph waveform obtained when an electrocardiograph is measured for a predetermined time (for example, 24 hours). When there is no noise, an electrocardiograph waveform obtained by measuring an electrocardiograph is constituted by a P wave, a QRS wave, and a T wave, for example, as illustrated in FIG. 5. The less the noise, the more the intervals between adjacent R waves (RR intervals) of the electrocardiograph waveform become the same, and the more the noise, the more the variation in the RR intervals becomes. Therefore, it can be determined that the higher the average value of the RR intervals, the less noise is present and the more stably the electrocardiograph is measured. When the average value of the RR interval is 0.5 or more, the electrocardiograph can be stably measured, and a highly reliable electrocardiograph can be obtained.

In addition, when the RR interval is analyzed from the electrocardiograph waveform, a generally used heart rate variability measure software (for example, RRI Analyzer, manufactured by Union Tool) or the like can be used.

In addition, the electrode pad 1 can have excellent followability to unevenness because the electrode 11 has an elongation at break of 50% to 600% and has moderate flexibility. Therefore, when the electrode 11 is attached to the lower surface 3a of the adhesive layer 3, the volumes of the gap formed between the electrode 11 and the adhesive layer 3 filled in the through hole 111 in the electrode 11 can be suppressed. Therefore, the electrode pad 1 can reduce the impedance between the electrode 11 and the skin 2a while maintaining the state of being attached to the adhesive layer 3. In addition, the electrode pad 1 can suppress the entry of external moisture, such as moisture of the subject 2 and sweat on the skin 2a, into the gap through the second conductive layer 12. Therefore, the electrode pad 1 can improve durability. Further, when the electrode 11 is provided on the upper surface 4a of the wiring 4, the occurrence of a gap between the electrode 11 and the wiring 4 can be reduced. Therefore, the electrical pad 1 can reliably transmit the electrical signal related to the biometric information transmitted from the skin 2a from the electrode 11 to the wiring 4.

The followability to unevenness of the electrode pad 1 can be evaluated from, for example, the volume of the gap when the electrode pad 1 expands and contracts. The volume of the gap can be obtained by using a general measurement method, and can be obtained by using a microscope such as a laser microscope, an optical microscope, or a scanning electron microscope (SEM). For example, the volume of the gap can be determined by determining the diameter of the gap when the gap is spherical voids, the median value of the short axis and the long axis of the void when the gap is elliptical voids, and the median value of the short side and the long side of the void when the gap is irregular.

The contact impedance with the skin 2a can be made 1 MΩ or less. When the contact impedance becomes 1 MΩ or less, for example, an electrocardiograph can be measured. The lower the contact impedance, the easier the measurement of electrocardiographs as biometric information. The contact impedance of the electrode pad 1 with the skin 2a is set to be 1 MΩ or less, so that the electrode pad 1 can be effectively used for a living body sensor for measuring electrocardiographs.

The polarization voltage of the electrode pad 1 can be set to 0.1 mV to 100 mV. This allows to acquire an electrical signal related to the biometric information and maintain the contact impedance at 1 MΩ or less, and thus makes it easy to measure electrocardiographs as the biometric information. Therefore, the electrode pad 1 can be effectively used for a living body sensor for measuring electrocardiographs.

In the electrode pad 1, a pseudoplastic fluid or a Bingham fluid can be used for the second conductive layer 12. Pseudoplastic fluids and Bingham fluids can have high viscosity when a force of a certain range of magnitude is applied. The second conductive layer 12 can be easily formed on the lower surface 11a of the electrode 11 by containing the pseudoplastic fluid or the Bingham fluid as the viscous fluid, and the generation of a gap between the electrode 11 and the second conductive layer 12 can be reduced. In addition, even if the skin 2a has small irregularities when the electrode pad 1 is attached to the skin 2a, the second conductive layer 12 can sufficiently follow the irregularities of the skin 2a, and the occurrence of a gap between the second conductive layer 12 and the skin 2a can be reliably reduced. Therefore, the electrode pad 1 can suppress the impedance between the electrode 11 and the skin 2a from increasing and varying more reliably.

In the electrode pad 1, the second conductive layer 12 can have a viscosity of 1 mPas to 100,000 mPas. Thus, when the electrode pad 1 is attached to the skin 2a, even if the skin 2a has small irregularities, the second conductive layer 12 can sufficiently follow the irregularities of the skin 2a, and the occurrence of a gap between the second conductive layer 12 and the skin 2a can be reliably reduced. Therefore, the electrode pad 1 can suppress the impedance between the skin 2a of the subject 2 and the second conductive layer 12 from increasing and varying more reliably.

The electrode pad 1 may contain one or more components selected from the group consisting of Cu, Na, Sn, Al, Si, K, Ag, and Ca as the metal of the metal chloride contained in the second conductive layer 12. The second conductive layer 12 can reliably exhibit conductivity by containing a metal chloride containing these metals. The second conductive layer 12 ensures the conductivity between the electrode 11 and the skin 2a, and thus the polarization voltage of the second conductive layer 12 can be reduced, and the increase and variation in impedance between the electrode 11 and the second conductive layer 12 can be suppressed. Therefore, the low impedance between the electrode 11 and the skin 2a can be maintained more reliably.

The impedance between the electrode 11 and the skin 2a can be maintained in a low state, and therefore the electrode pad 1 can be used for a living body sensor that acquires biometric information from a living body. When the electrode pad 1 is used for a living body sensor, the electrode pad 1 can use the electrode 11 as a bioelectrode for acquiring biometric information from the skin 2a. The electrode pad 1 can be suitably used as a living body sensor electrode pad of an attach-type living body sensor which is attached to the skin or the like of a living body and requires stable adhesion and high conductivity.

<Living Body Sensor>

A living body sensor including the electrode pad according to the present embodiment will be described. In the present embodiment, the living body sensor according to the present embodiment is an attachment type living body sensor that is attached to skin, which is a part of a living body, to measure biological information. With regard to the present embodiment, as an example, a case where the living body is a person will be described.

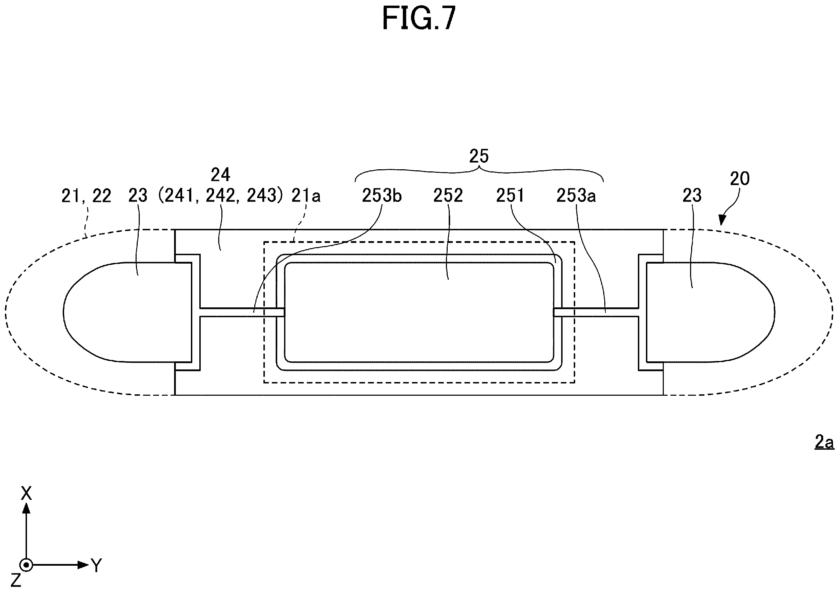

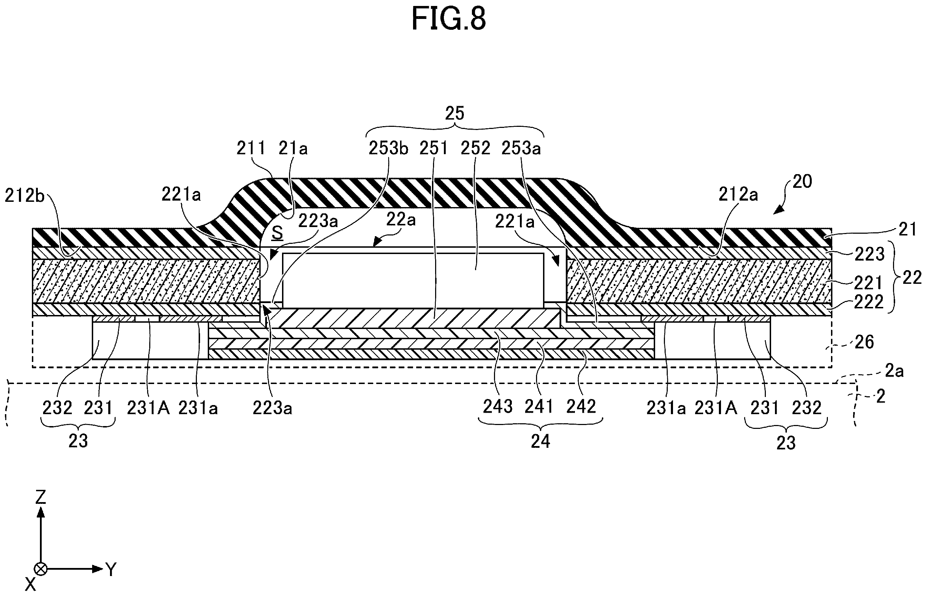

FIG. 6 is a perspective view illustrating a configuration of a living body sensor including the electrode pad according to the present embodiment. FIG. 7 is a plan view schematically illustrating the configuration of the living body sensor. FIG. 8 is a cross-sectional view taken along line I-I of FIG. 6.

As illustrated in FIGS. 6 and 7, the living body sensor 20 is a plate-like (sheet-like) member formed in a substantially elliptical shape in a plan view. As illustrated in FIG. 8, the living body sensor 20 includes a housing (cover member) 21, a foam sheet 22, electrode pads 23, a supporting adhesive sheet 24, and a sensor unit 25. The living body sensor 20 is formed by laminating the housing 21, the foam sheet 22, the electrode pads 23, and the supporting adhesive sheet 24 in this order from the housing 21 side toward the supporting adhesive sheet 24 side. The foam sheet 22, the electrodes 23, and the supporting adhesive sheet 24 are attached to the skin 2a of the subject 2, who is a living body, and the living body sensor 20 acquires a biological signal from the skin 2a via the electrodes 23. The electrode pad according to the present embodiment is used as the electrode pads 23.

The biological signal is, for example, an electric signal representing an electrocardiograph waveform, brain waves, a pulse, or the like.

The housing 21, the foam sheet 22, and the supporting adhesive sheet 24 have substantially the same outer shape in a plan view. The sensor unit 25 is provided on the supporting adhesive sheet 24 and is housed in a housing space S formed by the housing 21, the foam sheet 22, and the supporting adhesive sheet 24.

The living body sensor 20 may have a release paper 26 attached onto living-body-attachment-side surfaces of the foam sheet 22, the electrodes 23, and the supporting adhesive sheet 24 when not in use, and may be used by being attached to the skin 2a after the release paper 26 is peeled off when in use.

[Housing]

As illustrated in FIGS. 6 and 8, the housing 21 is located on the outermost side (+Z-axis direction) of the living body sensor 20 and is bonded to the upper surface of the foam sheet 22. The housing 21 includes a protruding portion 211 protruding in a substantially dome shape in a height direction (+Z-axis direction) in FIG. 1 at the center portion in a longitudinal direction (Y-axis direction), and flat portions 212a and 212b which are provided on both end sides in the longitudinal direction (Y-axis direction) of the housing 21. The upper surface and the lower surface of the protruding portion 211 and the upper surface and the lower surface of the flat portions 212a and 212b may be formed to be flat.

The housing 21 has a recess 21a formed in an indented shape from a living body side on an inner side (attachment side) of the protruding portion 211. From the inner side (attachment side) of the protruding portion 211, a housing space S for housing the sensor unit 25 is formed by the recess 21a formed on the inner surface of the protruding portion 211 and a hole 221a of the foam base 221.

As a material of forming the housing 21, for example, a flexible material such as silicone rubber, fluorine rubber, or urethane rubber can be used. In addition, the housing 21 can be formed by using a base resin such as polyethylene terephthalate (PET) as a support and laminating the material having flexibility on a surface of the support. By forming the housing 21 using the above-described material having flexibility or the like, the sensor unit 25 disposed in the housing space S of the housing 21 can be protected, and the impact applied to the living body sensor 20 from the upper surface side can be absorbed to reduce the impact applied to the sensor unit 25.

The thicknesses of the upper wall and the side walls of the protruding portion 211 may be thicker than the thicknesses of the flat portions 212a and 212b. Thus, the flexibility of the protruding portion 211 can be made lower than the flexibility of the flat portions 212a and 212b, and therefore the sensor unit 25 can be protected from an external force applied to the living body sensor 20.

The thicknesses of the upper wall and the side walls of the protruding portion 211 may be, for example, 1.5 mm to 3 mm, and the thicknesses of the flat portion 212a and the 212b may be, for example, 0.5 mm to 1 mm.

The thin flat portions 212a and 212b are more flexible than the protruding portion 211, and thus, when the living body sensor 20 is attached to the skin 2a, the thin flat portions 212a and 212b can be easily deformed following deformation of the skin 2a caused by body motion such as stretching, bending, or twisting. As a result, stresses applied to the flat portions 212a and 212b when the skin 2a is deformed can be moderated, and the living body sensor 20 can be prevented from being easily peeled off from the skin 2a.

Outer peripheral portions of the flat portion 212a and 212b have shapes whose thicknesses gradually decrease toward the ends. Accordingly, it is possible to further increase the flexibility of the outer peripheral portions of the flat portions 212a and 212b, and it is possible to improve a person's feeling of wearing when the living body sensor 20 is attached to the skin 2a compared to a case where the thicknesses of the outer peripheral portions of the flat portions 212a and 212b do not decrease.

The hardness (strength) of the housing 21 can be designed to be any appropriate value, and may be, for example, 40 to 70. When the hardness of the housing 21 is within the above-described preferable range, the foam sheet 22 can be deformed in accordance with the movement of the skin 2a without being affected by the housing 21 when the skin 2a is stretched by the body motion. The hardness refers to Shore A hardness.

[Foam Sheet]

As illustrated in FIG. 8, the foam sheet 22 is provided to be bonded to the lower surface of the housing 21. The foam sheet 22 has a through hole 22a at a position facing the protruding portion 211 of the housing 21. With the through hole 22a, the sensor unit 25 is stored in the housing space S formed by the recess 21a, the through hole 22a, and the supporting adhesive sheet 24 on the inner surface of the housing 21 without being blocked by the foam sheet 22.

As illustrated in FIG. 8, the foam sheet 22 includes a foam base (also referred to as a foam) 221, a base adhesive layer 222 provided on a surface of the foam base 221 on the living body side (-Z-axis direction), and a housing adhesive layer 223 provided on a surface of the foam base 221 on the housing 21 side (+Z-axis direction). The foam base 221 and the base adhesive layer 222 may be laminated and integrated to be used as a foaming attachment layer.

(Foam Base)

The foam base 221 has a porous structure and can be formed using a porous body having flexibility, waterproofness, and moisture permeability. As the porous body, for example, a foam material such as open cells, closed cells, or semi-closed cells, or the like can be used. Thus, water vapor generated from sweat or the like generated from the skin 2a to which the living body sensor 20 is attached can be released to the outside of the living body sensor 20 via the foam base 221.

The breaking elongation of the foam base 221 may be 30% to 500%. When the elongation at break of the foam base 221 is 30% or more, the living body sensor can follow the movement of the skin 2a, noise generated in an electrocardiographic waveform is reduced, and the subject is less likely to feel uncomfortable. When the elongation at break of the foam base 221 is 500% or less, the foam base 221 does not become too soft even if the volume of the pores formed inside the foam base 221 is large, and therefore, the shape of the foam base 221 can be maintained. In addition, since an entering of liquid such as water or sweat into the pores of the foam base 221 and a gap between the foam base 221 and the base adhesive layer 222 or the housing adhesive layer 223 can be suppressed, thereby a durability of the foam base 221 can be easily maintained. When the elongation at break of the foam base 221 is 30% to 500%, the living body sensor 20 is easily elongated in a state of being in contact with the skin 2a, and can maintain the state of being in contact with the skin 2a. Therefore, noise generated in the electrocardiographic waveform can be reduced, and discomfort given to the subject can be reduced. In addition, since the entry of liquid into the pores in the foam base 221 and the gap between the foam base 221 and the base adhesive layer 222 or the housing adhesive layer 223 can be suppressed, the durability of the foam base 221 can maintained.

The elongation at break of the foam base 221 refers to a ratio of the length of the foam base 221 in the long axial direction or the short axial direction at the time of breaking of the foam base 221 to the length of the foam base 221 in the long axial direction or the short axial direction before being pulled, as depicted in the following formula (1).

Elongation at break of the foam base 221 = (length of foam base 221 in the long axial direction or the short axial direction at a time of breaking) / (length of foam base 221 in the long axial direction or the short axial direction before being pulled)               (1).

The breaking elongation of the foam base 221 can be measured using a tensile tester (AGS-J, manufactured by Shimadzu Corporation). The tensile test conditions at this time can be set appropriately. For example, the tensile test can be performed with the foam base 221 having a width (the maximum width of the foam base 221 in the short axial direction) of 10 mm, a distance between a pair of jigs gripping both end portions of the foam base 221 being 50 mm, and the tensile strength of 300 mm/min.

The elongation at break of the foam base 221 may be an average value of the measured values of a plurality of (for example, three) foam bases 221.

In the present embodiment, the outer shape of the foam base 221 is an elliptical shape, and when the foam base 221 is attached to the skin 2a, the foam base 221 is most likely to expand and contract in the long axial direction. Therefore, the elongation at break of the foam base 221 is preferably the elongation at break in the long axial direction of the foam base 221. When the outer shape of the foam base 221 is rectangular, the elongation at break of the foam base 221 is preferably the elongation at break in the long-side direction of the foam base 221. When the outer shape of the foam base 221 is a square, the elongation at break of the foam base 221 may be the elongation at break in one side direction of the foam base 221. When the outer shape of the foam base 221 is circular, the elongation at break of the foam base 221 may be the elongation at break of a diameter of the foam base 221.

As a material of the foam base 221, for example, a thermoplastic resin such as a polyurethane-based resin, a polystyrene-based resin, a polyolefin-based resin, a silicone-based resin, an acrylic-based resin, a vinyl chloride-based resin, or a polyester-based resin can be used.

The thickness of the foam base 221 can be set appropriately, and can be, for example, 0.5 mm to 1.5 mm.

The foam base 221 has the hole 221a at a position facing the protruding portion 211 of the housing 21. Through holes 222a and 223a can be formed as a result of the base adhesive layer 222 and the housing adhesive layer 223 being formed on the area of the surface other than the area of the hole 221a of the foam base 221.

(Base Adhesive Layer)

As illustrated in FIG. 8, the base adhesive layer 222 is provided in such a manner as being attached to the lower surface of the foam base 221, and has a function of bonding the foam base 221 and the base 241 and bonding the foam base 221 and the electrode pads 23.

The base adhesive layer 222 preferably has moisture permeability. Water vapor or the like generated from the skin 2a to which the living body sensor 20 is attached can be released to the foam base 221 via the base material adhesive layer 222. Furthermore, since the foam base 221 has the porous structure as described above, water vapor can be released to the outside of the living body sensor 20 through the housing adhesive layer 223. As a result, it is possible to suppress accumulation of sweat or water vapor at the interface between the skin 2a on which the living body sensor 20 is attached and an attachment adhesive layer 242. As a result, it is possible to prevent the adhesive force of the base adhesive layer 222 from being weakened by the moisture accumulated at the interface between the skin 2a and the base adhesive layer 222, and thus, it is possible to prevent the living body sensor 20 from being peeled off from the skin 2a.

The moisture permeability of the base adhesive layer 222 may be in a range from 1 (g/m$^2$·day) to 10,000 (g/m$^2$·day). The moisture permeability of the base adhesive layer 222 is within the above range, such that when the attachment adhesive layer 242 is attached to the skin 2a, sweat or the like coming from the supporting adhesive sheet 24 can penetrate toward the outside, so that a burden to the skin 2a can be suppressed.

A material forming the base adhesive layer 222 is preferably a material having pressure-sensitive adhesiveness, and the same material as that of the attachment adhesive layer 242 can be used, and an acrylic pressure-sensitive adhesive is preferably used.

As the base adhesive layer 222, a double-sided adhesive tape formed of the above-described material can be used. It is possible to improve the waterproof property of the living body sensor 20 and to improve the adhesion strength with the housing 21 when the living body sensor 20 is formed from stacking the housing 21 on the base adhesive layer 222.

On the surface of the base adhesive layer 222, a wavy pattern (web pattern) may be formed in which adhesive provided portions in each of which an adhesive is present and adhesive not provided portions in each of which no adhesive is present are alternately formed. As the base adhesive layer 222, for example, a double-sided adhesive tape having a web pattern formed on its surface can be used. When the base adhesive layer 222 has the web pattern on the surface thereof, the adhesive can be made to be present on the protruding portions of the surface and the periphery thereof, and the adhesive can be prevented from being present on the indented portions of the surface and the periphery thereof. Therefore, since both the portions in each of which the adhesive is present and the portions in each of which the adhesive is not present are present on the surface of the base adhesive layer 222, the adhesive can be dotted on the surface of the base adhesive layer 222. The moisture permeability of the base adhesive layer 222 tends to increase as the thickness of the adhesive decreases. Therefore, since the web pattern is formed on the surface of the base adhesive layer 222 and the surface of the base adhesive layer 222 has the portions where the adhesive is partially thin, it is possible to improve the moisture permeability while maintaining the adhesive force as compared with a case where the web pattern is not formed.

Each of the widths of the adhesive provided portions and the adhesive not provided portions can be designed appropriately. For example, each of the widths of the adhesive provided portions is preferably 500 um to 1000 $\mu$m, and each of the widths of the adhesive not provided portions is preferably 1500 um to 5000 um. When the widths of the adhesive provided portions and the adhesion not provided portions each fall within the above-described preferable ranges, the base adhesive layer 222 can exhibit excellent moisture permeability while maintaining the adhesive force.

The thickness of the base adhesive layer 222 can be set appropriately, and is preferably 10 um to 300 um. When the thickness of the base adhesive layer 222 is within the above-described preferable range, the thickness of the living body sensor 20 can be reduced.

(Housing Adhesive Layer)

As illustrated in FIG. 8, the housing adhesive layer 223 is provided in a state of being attached to the upper surface of the foam base 221. The housing adhesive layer 223 is attached to the upper surface of the foam base 221 at a position corresponding to the flat portions at the attachment side (-Y-axis direction) of the housing 21, and has a function of bonding the foam base 221 and the housing 21.

As a material for forming the housing adhesive layer 223, for example, a silicon-based adhesive tape or the like can be used.

The thicknesses of the housing adhesive layer 223 can be set appropriately, and may be, for example, in a range from 10 um to 300 $\mu$m.

(Electrode Pad)

As shown in FIG. 8, the electrode pads 23 are attached to the lower surface of the base adhesive layer 222 on the attachment side (-Z-axis direction) in a state in which parts of the electrode pads 23 at the sensor unit 25 sides are connected to wirings 25a and 25b and are sandwiched between the base adhesive layer 222 and a sensor adhesive layer 243. Portions of the electrode pads 23 that are not sandwiched between the base adhesive layer 222 and the sensor adhesive layer 243 come into contact with the living body. When the living body sensor 20 is attached to the skin 2a, the electrode pads 23 come into contact with the skin 2a, and thus a biological signal can be detected.

The electrode pads 23 may be embedded in the base 241 in a state of being exposed so as to be able to contact the skin 2a.

The electrode pad 1 according to the present embodiment is used as the electrode pads 23. The electrode pad 23 includes an electrode 231 and a second conductive layer 232. The electrode 231 and the second conductive layer 232 are similar to the electrode 11 and the second conductive layer 12 of the electrode pad 1, and thus, the details thereof will be omitted.

The electrodes 231 preferably have a through hole 231A in the main surface (lower surface) 231a in contact with the second conductive layer 232. Thus, the base adhesive layer 222 can be exposed from the through hole 231A to the second conductive layer 232 side in a state where the electrode pads 23 are attached to the base adhesive layer 222. Therefore, the adhesion between the second conductive layer 232 and the base adhesive layer 222 can be increased.

The height of the second conductive layer 232 can be set to any height as appropriate, but is preferably set to be substantially equal to the attachment surface of the attachment adhesive layer 242 of the supporting adhesive sheet 24, in that the foam sheet 22, the electrodes pads 23, and the supporting adhesive sheet 24 form the attachment surface with the skin 2a.

[Supporting Adhesive Sheet]

As illustrated in FIG. 8, the supporting adhesive sheet 24 includes the base 241, the attachment adhesive layer 242, and the sensor adhesive layer 243. In the supporting adhesive sheet 24, the base 241, the attachment adhesive layer 242, and the sensor adhesive layer 243 may be formed in the same shape in a plan view.

(Base)

As illustrated in FIG. 7, the base 241 may be formed in a substantially rectangular shape. The contour shapes on both sides in the width direction (X-axis direction) of the base 241 is substantially the same as the contour shapes on both sides in the width direction (X-axis direction) of the housing 21 and the foam sheet 22. The length (Y-axis direction) of the base 241 is formed to be shorter than the length (Y-axis direction) of the housing 21 and the foam sheet 22. Both ends of the supporting adhesive sheet 24 in the longitudinal direction are at positions where the wirings 253a and 253b of the sensor unit 25 are sandwiched between the supporting adhesive sheet 24 and the foam sheet 22, and are at positions of overlapping partially with the electrode pads 23.

The sensor adhesive layer 243 is provided on the upper surface of the base 241, and the sensor adhesive layer 243 is provided on the attachment surface of the base 241. The sensor adhesive layer 243, the base adhesive layer 222 of the foam sheet 22 that further extends from both ends in the longitudinal direction of the supporting adhesive sheet 24, and the second conductive layer 232 form an attachment surface to the skin 2a. Accordingly, waterproofness and moisture permeability are different and adhesiveness is different depending on the position of the attachment surface, but, in terms of the entire living body sensor 20, the adhesiveness in the attachment surface corresponding to the foam sheet 22 greatly affects the adhesive performance with respect to the skin 2a.

The base 241 can be formed using a flexible resin having appropriate stretchability, flexibility, and toughness. Examples of a material of forming the base 241 include thermoplastic resins such as polyester resins such as polyethylene terephthalate (PET), polybutylene terephthalate, polytrimethylene terephthalate, polyethylene naphthalate, and polybutylene naphthalate; acrylic resins such as polyacrylic acid, polymethacrylic acid, polymethyl acrylate, polymethyl methacrylate (PMMA), polyethyl methacrylate, and polybutyl acrylate; polyolefin resins such as polyethylene and polypropylene; polystyrene resins such as polystyrene, imide-modified polystyrene, acrylonitrile-butadiene-styrene (ABS) resin, imide-modified ABS resin, styrene-acrylonitrile copolymer (SAN) resin, and acrylonitrile-ethylene-propylene-diene-styrene (AES) resin; polyimide resins; polyurethane resins; silicone resins; and polyvinyl chloride resins such as polyvinyl chloride and vinyl chloride-vinyl acetate copolymer resins. Among them, the polyolefin resins and PET are preferable. These thermoplastic resins have waterproof properties (low moisture permeability). Therefore, by forming the base 241 using any of these thermoplastic resins, it is possible to prevent intrusion of sweat or water vapor generated from the skin 2a into the flexible substrate 251 side of the sensor unit 25 through the base 241 in a state where the living body sensor 20 is attached to the skin 2a of a subject 2.

The base 241 is preferably formed in a flat plate shape because the sensor unit 25 is provided on the upper surface of the base 241.

The thickness of the base 241 can be appropriately selected. For example, the thickness is preferably 1 um to 300 $\mu$m.

(Attachment Adhesive Layer)

As illustrated in FIG. 8, the attachment adhesive layer 242 is provided on the lower surface of the base 241 on the attachment side (-Z-axis direction), and is a layer that comes into contact with the subject 2.

The attachment adhesive layer 242 preferably has pressure-sensitive adhesiveness. When the attachment adhesive layer 242 has pressure-sensitive adhesiveness, the living body sensor 20 can be easily made to be attached to the skin 2a of a living body as a result of the living body sensor 20 being pressed onto the skin 2a.

The material of the attachment adhesive layer 242 is not particularly limited as long as it is a material having pressure-sensitive adhesiveness, and examples thereof include a material having biocompatibility. Examples of the material of forming the attachment adhesive layer 242 include an acrylic pressure-sensitive adhesive and a silicone pressure-sensitive adhesive. Preferably, the acrylic pressure-sensitive adhesive is used.

The acrylic pressure-sensitive adhesive preferably contains an acrylic polymer as a principal ingredient. The acrylic polymer can function as a pressure-sensitive adhesive ingredient. As the acrylic polymer, a polymer obtained by polymerizing a monomer ingredient containing a (meth)acrylic acid ester such as isononyl acrylate or methoxyethyl acrylate as a principle ingredient and a monomer copolymerizable with the (meth)acrylic acid ester such as acrylic acid as an optional ingredient can be used.

The acrylic pressure-sensitive adhesive preferably further contains a carboxylic acid ester. The carboxylic acid ester functions as a pressure-sensitive adhesive force adjusting agent that reduces the pressure-sensitive adhesive force of the acrylic polymer to adjust the pressure-sensitive adhesive force of the attachment adhesive layer 242. As the carboxylic acid ester, a carboxylic acid ester compatible with acrylic polymer can be used. As the carboxylic acid ester, glyceryl tri-fatty acid or the like can be used.

The acrylic pressure-sensitive adhesive may contain a crosslinking agent if necessary. The crosslinking agent is a crosslinking ingredient that crosslinks the acrylic polymer. Examples of the crosslinking agent include polyisocyanate compounds (polyfunctional isocyanate compounds), epoxy compounds, melamine compounds, peroxide compounds, urea compounds, metal alkoxide compounds, metal chelate compounds, metal salt compounds, carbodiimide compounds, oxazoline compounds, aziridine compounds, and amine compounds. Among them, the polyisocyanate compounds are preferable. These crosslinking agents may be used alone or in combination.

The attachment adhesive layer 242 preferably has excellent biocompatibility. For example, when the attachment adhesive layer 242 is subjected to a keratin peeling test, the keratin peeling area ratio is preferably 0% to 50%. If the keratin peeling area ratio is in the range of 0% to 50%, a burden to the skin 2a can be suppressed when the attachment adhesive layer 242 is attached to the skin 2a.

The attachment adhesive layer 242 preferably has moisture permeability. Water vapor or the like generated from the skin 2a to which the living body sensor 20 is attached is allowed to pass to the foam sheet 22 side through the attachment adhesive layer 242. Furthermore, since the foam sheet 22 has a porous structure as will be described later, water vapor is allowed to pass to the outside of the living body sensor 20 through the attachment adhesive layer 242. As a result, it is possible to suppress accumulation of sweat or water vapor at the interface between the skin 2a on which the living body sensor 20 is attached and the attachment adhesive layer 242. As a result, the adhesive force of the attachment adhesive layer 242 is prevented from being weakened by moisture accumulated at the interface between the skin 2a and the attachment adhesive layer 242, and therefore, the living body sensor 20 can be prevented from being peeled off from the skin.

The moisture permeability of the attachment adhesive layer 242 is preferably, for example, 300 (g/m$^2$·day) to 10,000 (g/m$^2$·day). When the moisture permeability of the attachment adhesive layer 242 is within the above-described preferable range, even if the attachment adhesive layer 242 is attached to the skin 2a, the sweat or the like generated from the skin 2a can be appropriately allowed to pass from the base 241 to the outside, and thus the burden on the skin 2a can be reduced.

The thickness of the attachment adhesive layer 242 can be appropriately and arbitrarily selected, and is preferably 10 um to 300 um. When the thickness of the attachment adhesive layer 242 is within the above-described preferable range, the thickness of the living body sensor 20 can be reduced.

(Sensor Adhesive Layer)

As illustrated in FIG. 8, the sensor adhesive layer 243 is provided on the upper surface of the base 241 at the housing 21 side (+Z-axis direction), and is a layer to which the sensor unit 25 is bonded. The sensor adhesive layer 243 can be made of the same material as that of the attachment adhesive layer 242, and thus will not be described in detail here. Note that the sensor adhesive layer 243 is not necessarily provided, and instead of the sensor adhesive layer 243, an adhesive may be provided on a part or the entirety of the base 241.

(Sensor Unit)

As illustrated in FIGS. 7 and 8, the sensor unit 25 includes a flexible substrate 251 on which various components for acquiring biometric information are mounted, a sensor main body 52, and wirings 253a and 253b which are connected to the sensor main body 52 in the longitudinal direction.

The flexible substrate 251 is a resin substrate. The sensor main body 52 and the wirings 253a and 253b are integrally formed on the flexible substrate 251.

The sensor main body 52 is provided on the flexible substrate 251 and includes a control unit and a battery (not illustrated).

The control unit (not illustrated) includes various components mounted on the flexible substrate 251, such as a CPU and an integrated circuit that process a biological signal acquired from a living body to generate biological signal data, a switch that activates the living body sensor 20, a flash memory that stores a biological signal, and a light emitting element.

The control unit (not illustrated) operates with power supplied from a battery (not illustrated). The control unit (not illustrated) performs wired or wireless signal transmission to external devices such as an operation check device that checks an initial operation, a reading device that reads biological information from the living body sensor 20, and the like.

The battery (not illustrated) supplies electric power to an integrated circuit or the like mounted on the control unit (not illustrated). As the battery, a known battery can be used. As the battery, for example, a coin-type battery such as a CR2025 can be used.

As illustrated in FIGS. 7 and 8, the wirings 253a and 253b are connected at one end to the electrodes 231 of the electrode pads 23. The other ends of the wirings 253a and 253b are connected to the sensor main body 52. The wirings 253a and 253b may be formed on either the front side or the back side of the flexible substrate 251.

As illustrated in FIG. 8, the living body sensor 20 is such that, in order to protect the base 241 and the electrode pads 23, it is preferable that release paper 26 is attached to the attachment surface side (-Z-axis direction) until the living body sensor 20 is attached to skin 2a. Thus, as a result of the release paper 26 being peeled off from the base 241 and the electrode pads 23 at a time of use, the adhesive strength of the base 241 can be maintained until then.

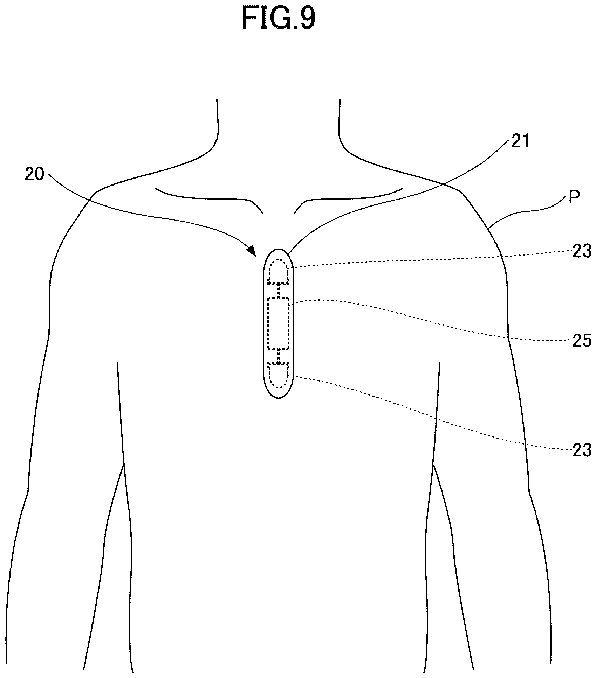

FIG. 9 is an explanatory view illustrating a state in which the living body sensor 20 of FIG. 1 is attached to a chest of a living body P. For example, the living body sensor 20 is attached to the skin of the subject P in such a manner that the longitudinal direction (Y-axis direction) is aligned with the sternum of the subject P, one electrode 23 is at the upper side, and the other electrode 23 is at the lower side. The living body sensor 20 obtains a biological signal such as an electrocardiographic signal from the subject P through the electrodes 23 in a state where the electrodes 23 are pressed onto the skin of the subject P as a result of being attached to the skin of the subject P with the foam sheet 22, the electrode pads 23, and the supporting adhesive sheet 24. The living body sensor 20 stores the obtained biological signal data in a nonvolatile memory such as a flash memory mounted in the sensor unit 25.

A method of manufacturing the living body sensor 20 is not particularly limited, and the living body sensor 20 can be manufactured by any appropriate method. An example of a method of manufacturing the living body sensor 20 will be described.

The housing 21, the foam sheet 22, the electrode pads 23, the supporting adhesive sheet 24, and the sensor unit 25 illustrated in FIG. 6 are prepared. The housing 21, the foam sheet 22, and the supporting adhesive sheet 24 are not particularly limited as long as they can be manufactured by these methods, and these products can be manufactured in any manufacturing method.

As described above, the electrode pads 23 can be manufactured by any appropriate manufacturing method without any particular limitation as long as the electrode pads 23 can be manufactured by the method, similarly to the method for manufacturing the electrode pads according to the present embodiment.

After the housing 21, the foam sheet 22, the electrode pads 23, the supporting adhesive sheet 24, and the sensor unit 25 constituting the living body sensor 20 illustrated in FIGS. 6 to 8 are prepared, the sensor unit 25 is placed on the supporting adhesive sheet 24. Thereafter, the housing 21, the foam sheet 22, the electrode pads 23, and the supporting adhesive sheet 24 are laminated in this order from the housing 21 side toward the supporting adhesive sheet 24 side. Thus, the living body sensor 20 illustrated in FIG. 6 is obtained.

The release paper 26 may be attached to the living-body-attachment-side surfaces of the foam sheet 22 and the supporting adhesive sheet 24.

Alternatively, in the electrode pads 23, the electrodes 231 may be first attached to the supporting adhesive sheet 24, and then the second conductive layers 232 may be formed only on the lower surface 231a of the electrodes 231 or on the lower surfaces 231a of the electrodes 231 and the supporting adhesive sheet 24 around the lower surfaces 231a of the electrodes 231 to manufacture the living body sensor 20. That is, after the housing 21, the foam sheet 22, the electrodes 231, the supporting adhesive sheet 24, and the sensor unit 25 are prepared, the sensor unit 25 is placed on the supporting adhesive sheet 24. Thereafter, the housing 21, the foam sheet 22, the electrodes 231, and the supporting adhesive sheet 24 are laminated in this order from the housing 21 side toward the supporting adhesive sheet 24 side. Thereafter, a composition for forming a second conductive layer (second conductive layer forming paste) containing a metal chloride and a viscous fluid is applied only to the lower surfaces 231a of the electrodes 231 or to the lower surfaces 231a of the electrodes 231 and the supporting adhesive sheet 24 around the lower surface 231a, and is dried to form the second conductive layer 232. Thus, the living body sensor 20 is obtained.

As described above, the living body sensor 20 includes the base adhesive layer 222 and the electrode pads 23, and the electrode pad 1 according to the present embodiment is used as the electrode pads 23. Thus, the living body sensor 20 can suppress an increase and a variation in impedance between the skin 2a and the electrodes 231, and thus can maintain low impedance between the electrodes 231 and the skin 2a. When the living body sensor 20 is used for measuring an electrocardiograph, the living body sensor 20 can suppress an increase and a variation in noise generated in the electrocardiograph, and thus can stably measure the electrocardiograph for a long time.

That is, since the electrodes 231 of the living body sensor 20 have an elongation at break of 50% to 600% and have high stretchability as described above, even when the second conductive layer 232 is deformed by deformation of the skin 2a due to body motion of the subject 2, the electrodes 231 are flexibly deformed with respect to the deformation of the second conductive layer 232 and can maintain the bonded state. Therefore, when the living body sensor 20 is used by being attached to the skin 2a, even if the skin 2a is deformed by body motion, clothes rubbing, or the like due to walking, ascending, and descending of a staircase, or the like of the subject 2, the electrodes 23 are deformed following the movement of the skin 2a, and can maintain the contact state with the skin 2a and also maintain the contact state with the base adhesive layer 222. As described above, the second conductive layer 232 contains 0.1% by mass to 40% by mass of a metal chloride and has conductivity, and thus, it is possible to secure conduction between the skin 2a and the electrodes 231. In addition, since the polarization voltage generated by the difference in charge transfer between the skin 2a and the electrodes 231 can be reduced and the charge transfer between the skin 2a and the electrodes 231 can be assisted, the potential difference between the skin 2a and the electrodes 231 can be reduced and the skin 2a and the electrodes 11 can be easily conducted. Furthermore, since the second conductive layer 232 is a hydrated material having high fluidity, the second conductive layer 232 can be deformed to sufficiently follow small irregularities of the skin 2a, and the occurrence of a gap between the second conductive layer 232 and the skin 2a can be reduced. Even if the skin 2a moves due to the body motion of the subject 2 or the like when the living body sensor 20 is used by being attached to the skin 2a, the second conductive layer 232 deforms following the movement of the skin 2a, and can maintain the contact state with the skin 2a while reducing the occurrence gap between the second conductive layer 232 and the skin 2a. Therefore, the living body sensor 20 can suppress an increase and a variation in impedance between the electrodes 231 and the skin 2a and maintain the low impedance between the electrodes 231 and the skin 2a.

The living body sensor 20 can stably detect the electric signal related to the biometric information of the subject 2 by the electrodes 231 while maintaining the contact with the skin 2a by the electrodes 23, and thus can suppress an increase in noise generated when measuring the biometric information of an electrocardiograph or the like. Therefore, the living body sensor 20 can stably measure the biometric information from the skin 2a with high accuracy even when the subject moves by exercise or the like during use.

For example, the living body sensor 20 can suppress an increase in noise generated when the electrocardiograph is measured, and thus can maintain the average value of the RR interval of the electrocardiograph to be high, for example, 0.5 or more.

In addition, the living body sensor 20 can have excellent followability to unevenness by the electrodes 231 of the electrodes pads 23 having an elongation at break of 50% to 600% and having appropriate flexibility, and thus can suppress the volumes of gap generated between the electrodes 231 and the base adhesive layers 222 filled in the through holes 231A in the electrodes 231. Therefore, the living body sensor 20 can reduce the impedance between the electrodes 231 and the skin 2a while maintaining the state in which the electrodes pads 23 are attached to the sensor adhesive layer 243. The second conductive layer 232 is also disposed between the lower surface 231a of the electrode 231 and the side surface of the sensor adhesive layer 243, and can further suppress the volumes of the gap generated between the electrodes 231 and the base adhesive layers 222 filled in the through holes 231A of the electrodes 231 and between the lower surface 231a of the electrodes 231 and the side surface of the sensor adhesive layer 243. Therefore, the living body sensor 20 can suppress the entry of external moisture, such as moisture of the subject 2 and sweat on the skin 2a, into the gap through the second conductive layer 232, and thus can improve durability. Furthermore, even if the electrodes 23 are subjected to vibration, deformation, or the like due to body motion caused by walking, ascending or descending of a staircase, or the like of the subject 2, deformation of the skin 2a caused by clothes rubbing, or the like, it is possible to reduce the occurrence of a gap between the electrodes 231 and the wirings 253a and 253b. Therefore, the living body sensor 20 can reliably transmit the electric signal related to the biometric information transmitted from the skin 2a from the electrodes 231 to the wirings 253a and 253b, in the electrode pads 23.

In the living body sensor 20, the second conductive layers 232 of the electrode pads 23 can be provided to the periphery of the electrode pads 23. Thus, the second conductive layers 232 can further cover the side surfaces of the electrodes 231 and the lower surface of the base adhesive layers 222, and thus can suppress the formation of a gap between the side surfaces of the electrodes 231 and the lower surface of the base adhesive layers 222. Therefore, the living body sensor 20 can suppress the entry of external moisture into the gap via the second conductive layers 232, and thus can further enhance the durability.

The living body sensor 20 includes the base 241 and the attachment adhesive layer 242, and the attachment surface to the skin 2a can be formed in a substantially flat state by the base adhesive layer 222, the second conductive layer 232, and the attachment adhesive layer 242. The base adhesive layer 222, the attachment adhesive layer 242, and the second conductive layer 232 are all adhesive, and therefore, even when the electrodes pads 23 are attached to the base adhesive layer 222, the areas to be attached to the skin 2a can be reliably secured. This can prevent the living body sensor 20 from peeling off from the skin 2a, and thus can maintain the state where the electrodes 23 are stably attached to the skin 2a. Therefore, even when the living body sensor 20 is attached to the skin 2a of the subject for a long period of time, the living body sensor 20 can reliably detect the electric signal related to the biometric information from the skin 2a during use.

In the living body sensor 20, the through holes 231A are provided in the electrodes 231, and the base adhesive layers 222 can be exposed from the through holes 231A. If the base adhesive layers 222 are exposed to the attached side via the through holes 231A, it is possible to increase the adhesion between the electrode pads 23 and the base adhesive layers 222. Therefore, the living body sensor 20 can more reliably maintain the state of being attached to the skin 2a in a state where the electrodes pads 23 are attached to the base adhesive layers 222.

In this way, the living body sensor 20 can be effectively used as an attached-type living body sensor that is used by being attached to the skin 2a or the like of the subject 2. As described above, the living body sensor 20 can measure the biometric information from the skin 2a stably for a long period of time during use. Therefore, the living body sensor 20 can be suitably used for, for example, wearable devices for healthcare which are attached to the skin or the like of a living body, have high detection sensitivity of electrocardiographs, and are required to have a high effect of suppressing noise generated in electrocardiographs.

EXAMPLES

Hereinafter, the embodiment will be described more specifically with reference to Examples and Comparative Examples, but the embodiment is not limited to these Examples and Comparative Examples.

<Preparation of Electrode Sheet>

[Preparation of Electrode Sheet 1]

(Preparation of Conductive Composition Aqueous Solution)

0.38 parts by mass of PEDOT/PSS pellets ("Orgacon DRY", manufactured by Japan Agfa Materials) as a conductive polymer, 10.00 parts by mass of an aqueous solution containing modified polyvinyl alcohol (modified PVA) (modified polyvinyl alcohol concentration: 10%, "Gohsenex Z-410", manufactured by The Nippon Synthetic Chemical Industry Co., Ltd.) as a binder resin, 2.00 parts by mass of glycerin (manufactured by Wako Pure Chemical Industries, Ltd.) as a plasticizer, and 1.60 parts by mass of 2-propanol (IPA) and 4.40 parts by mass of water as solvents were added to an ultrasonic bath. Then, the aqueous solution containing these components was mixed for 30 minutes in the ultrasonic bath to prepare a uniform conductive composition aqueous solution 1.

Since the concentration of the modified PVA in the aqueous solution containing the modified PVA is about 10%, the content of the modified PVA in the conductive composition aqueous solution 1 is 1.00 parts by mass. The remainder is the solvent in the conductive composition aqueous solution 1.

The contents of the conductive polymer, the binder resin, and the plasticizer were 11.2 parts by mass, 29.6 parts by mass, and 59.2 parts by mass, respectively, with respect to 100.0 parts by mass of the conductive composition.

(Preparation of Electrode Sheet)

The prepared conductive composition aqueous solution 1 was applied onto a polyethylene terephthalate (PET) film using an applicator. Thereafter, the PET film coated with the conductive composition aqueous solution 1 was transported to a drying oven (SPHH-201, manufactured by ESPEC Corp.), and the conductive composition aqueous solution 1 was heated and dried at 135°C for 3 minutes, thereby producing a cured product of the conductive composition. The cured product was punched (pressed) into a desired shape to be formed into a sheet shape, and thus an electrode sheet 1 as a first conductive layer was produced.

The contents of the conductive polymer, the binder resin, and the plasticizer contained in the electrode sheet 1 are the same as those of the conductive composition.

(Measurement of Elongation at break)

A sample size of the sheet 1 was 10 mm in short side × 70 mm in long side × 0.5 mm in thickness. The sample of the electrode sheet 1 was pulled in the long axial direction thereof under the following tensile test conditions using a tensile tester (AGS-J, manufactured by Shimadzu Corporation), and the elongation at break in the long axial direction of the electrode sheet 1 when the electrode sheet 1 was broken was measured and evaluated based on the following evaluation criteria.

(Tensile Test Conditions)

Maximum width of the short axial direction of the sheet 1: 10 mm
Distance between jigs for placing the electrode sheet 1: 50 mm
Tensile strength: 300 mm/min

((Evaluation Criteria))

A: The elongation at break is 50% to 600%.
B: The elongation at break is less than 50%.
C: The elongation at break exceeds 600%.

[Preparation of Electrode Sheet 2]

A conductive composition aqueous solution 2 was prepared in the same manner as in the preparation of the conductive composition aqueous solution 1, except that the aqueous solution containing the modified PVA used in the preparation of the conductive composition aqueous solution 1 was not used in the electrode sheet 1. Then, the same procedure as in the production of the electrode sheet 1 was carried out using the conductive composition aqueous solution 2 to produce an electrode sheet 2.

[Preparation of Electrode Sheet 3]

A conductive composition aqueous solution 3 was prepared in the same manner as in the preparation of the conductive composition aqueous solution 1, except that the modified PVA used in the preparation of the conductive composition aqueous solution 1 was used as it was in the electrode sheet 1. Then, the same procedure as in the production of the electrode sheet 1 was carried out using the conductive composition aqueous solution 3 to produce an electrode sheet 3.

[Preparation of Electrode Sheet 4]

An electrode sheet 4 was produced by changing the electrode sheet 1 to a Cu film.

[Preparation of Electrode Sheet 5]

A conductive composition aqueous solution 4 was prepared in the same manner as in the preparation of the conductive composition aqueous solution 1, except that the content of glycerin used in the preparation of the conductive composition aqueous solution 1 was changed from 2.0 g to 1.50 g in the electrode sheet 1. Then, an electrode sheet 5 was produced in the same manner as in the production of the electrode sheet 1 using the conductive composition aqueous solution 4.

[Preparation of Electrode Sheet 6]

A conductive composition aqueous solution 5 was prepared in the same manner as in the preparation of the conductive composition aqueous solution 1, except that the content of glycerin used in the preparation of the conductive composition aqueous solution 1 was changed from 2.0 g to 0.10 g in the electrode sheet 1. Then, an electrode sheet 6 was produced in the same manner as in the production of the electrode sheet 1 using the conductive composition aqueous solution 5.

[Preparation of Electrode Sheet 7]

A conductive composition aqueous solution 6 was prepared in the same manner as in the preparation of the conductive composition aqueous solution 1, except that the content of glycerin used in the preparation of the conductive composition aqueous solution 1 was changed from 2.0 g to 6.00 g in the electrode sheet 1. Then, an electrode sheet 7 was produced in the same manner as in the production of the electrode sheet 1 using the conductive composition aqueous solution 6.

The content of each component contained in the conductive composition aqueous solution and the solvent used for forming the electrode sheet, the kind of metal, and the elongation at break of the electrode sheet are indicated in Table 1. The electrode sheets 1, 2, 4, 6, and 8 were all evaluated as A in the elongation at break, but among these, the electrode sheet 2 had particularly excellent elongation at break.

[Table 1]

| | | Components (parts by mass) | | | | | | | | |
|---|---|---|---|---|---|---|---|---|---|---|
| | | Conductive composition | | | | | Solvents | | Cu | Elongation at break [%] |
| | | PEDOT/PSS | Binder resin | Plasticizer | Total | Remainder | 2-propanol | Water | | |
| Electrode sheet 1 | Conductive composition aqueous solution 1 | 0.38 (11.2) | 1.00 (29.6) | 2.00 (59.2) | 3.38 (100.0) | 96.62 (-) | 1.60 | 4.40 | - | A |
| Electrode sheet 2 | Conductive composition aqueous solution 2 | 0.38 (16.0) | 0.00 (0.0) | 2.00 (84.0) | 2.38 (100.0) | 97.62 (-) | 1.60 | 4.40 | - | A |
| Electrode sheet 3 | Conductive composition aqueous solution 3 | 0.38 (3.10) | 10.00 (80.8) | 2.00 (16.2) | 12.38 (100.0) | 87.62 (-) | 1.60 | 4.40 | - | B |
| Electrode sheet 4 | Conductive composition aqueous solution 1 | 0.38 (13.2) | 1.00 (34.7) | 1.50 (52.1) | 2.88 (100.0) | 97.12 (-) | 1.60 | 4.40 | - | A |
| Electrode sheet 5 | - | - | - | - | - | - | - | - | 100 | C |
| Electrode sheet 6 | Conductive composition aqueous solution 4 | 0.38 (13.2) | 1.00 (34.7) | 1.50 (52.1) | 2.88 (100.0) | 97.12 (-) | 1.60 | 4.40 | - | A |
| Electrode sheet 7 | Conductive composition aqueous solution 5 | 0.38 (25.7) | 1.00 (67.6) | 0.10 (6.8) | 1.48 (100.0) | 98.52 (-) | 1.60 | 4.40 | - | C |
| Electrode sheet 8 | Conductive composition aqueous solution 6 | 0.38 (5.1) | 1.00 (13.2) | 6.00 (81.3) | 7.38 (100.0) | 92.62 (-) | 1.60 | 4.40 | - | A |

<Preparation of Second Conductive Layer>

[Preparation of Second Conductive Layer 1]

A second conductive layer 1 was prepared using 4.4 parts by mass of an aqueous solution (NaCl concentration: 2%) containing NaCl, polyethylene glycol, glyceryl, paraben, mineral oil, and polyoxyethylene stearate. The viscosity of the second conductive layer 1 was 727 mPas, the concentration was 0.5 wt%, and the polarization voltage was 86 mV. The viscosity and polarization voltage were measured as follows.

(Viscosity)

The viscosity was measured using a viscosity measuring device (rheometer (HAAKE MARS, manufactured by Thermo Fisher Scientific Inc.)) under the following measurement conditions.

((Measurement Conditions))

Measurement mode: hysteresis loop
Measurement jig: parallel plate (35 mm in diameters)
Gap: 0.5 mm
Measurement temperature: 30°C
Frequency: 60 Hz

[Preparation of Second Conductive Layer 2]

A second conductive layer 2 was prepared in the same manner as the second conductive layer 1, except that the aqueous solution containing NaCl was changed to an aqueous solution containing a KCl aqueous solution (KCl concentration: 200). The viscosity of the second conductive layer 2 was 2352 mPas, the concentration was 5 wt%, and the polarization voltage was 44 mV.

[Preparation of Second Conductive Layer 3]

A second conductive layer 3 was prepared in the same manner as the second conductive layer 1, except that the aqueous solution containing NaCl was changed to an aqueous solution containing a CaCl aqueous solution (CaCl concentration: 50%). The second conductive layer 3 had a viscosity of 18952 mPas, a concentration of 12.5 wt%, and a polarization voltage of 11 mV.

[Preparation of Second Conductive Layer 4]

A second conductive layer 4 was prepared in the same manner as the second conductive layer 1, except that the concentration of the aqueous solution containing NaCl was changed so that the viscosity was 0.55 mPas, the concentration was 0.9 wt%, and the polarization voltage was 131 mV.

[Preparation of Second Conductive Layer 5]

A second conductive layer 5 was prepared in the same manner as the second conductive layer 1, except that the concentration of the aqueous solution containing NaCl was changed so that the viscosity was 120836 mPas, the concentration was 0.5 wt%, and the polarization voltage was 213 mV.

[Preparation of Second Conductive Layer 6]

A second conductive layer 6 was prepared in the same manner as the second conductive layer 1, except that the aqueous solution containing NaCl was changed to water. The viscosity of the second conductive layer 6 was 914 mPas, the concentration was 0.0 wt%, and the polarization voltage was 248 mV.

[Preparation of Second Conductive Layer 7]

A second conductive layer 7 was prepared in the same manner as the second conductive layer 1, except that the concentration of the aqueous solution containing NaCl was changed so that the viscosity was 727 mPas, the concentration was 0.5 wt%, and the polarization voltage was 275 mV.

Table 2 indicates the metal chloride used for forming the second conductive layer, and the viscosity and concentration of the second conductive layer were also indicated in Table 2.

[Table 2]

|  | Metal chloride | Viscosity [mPas] | Concentration [w%] |
|---|---|---|---|
| Second conductive layer 1 | NaCl | 727 | 0.5 |
| Second conductive layer 2 | KCl | 2352 | 5.0 |
| Second conductive layer 3 | CaCl | 18952 | 12.5 |
| Second conductive layer 4 | NaCl | 0.55 | 0.9 |
| Second conductive layer 5 | NaCl | 120836 | 0.5 |
| Second conductive layer 6 | NaCl | 914 | 0.0 |
| Second conductive layer 7 | NaCl | 727 | 0.5 |

<Preparation and Evaluation of Electrode Pad>

[Example 1]

(Preparation of Electrode Pad)

The second conductive layer 1 was formed on the main surface of the electrode sheet 1 to prepare an electrode pad A.

(Evaluation of Electrode Pad)

The polarization voltage, impedance, followability to unevenness, average value of RR intervals, and presence or absence of peeling of the electrode pad were evaluated.

1. Polarization Voltage

The electrode pad A thus prepared was assumed to be the electrode sheet 1 immersed in physiological saline (NaCl concentration: 0.9%, 4.4 parts by mass). The polarization voltage of the electrode pad was measured by immersing a silver chloride electrode as a reference electrode in the physiological saline solution in addition to the electrode sheet 1, and measuring the potential difference between the electrode sheet 1 and the silver chloride electrode. The measured potential difference was assumed as the polarization voltage of the electrode pad A.

2. Measurement of Impedance

(1) Preparation of Adherend

Pig skin (Yucatan micropig (YMP) skin set, Charles River Laboratories Japan, Inc.) frozen at -80°C was naturally thawed and pretreated to remove subcutaneous fat. Then, the pre-treated pig skin was cut into 30 mm × 50 mm × 5 mm. Cut pig skin was used as the adherend.

(2) Preparation of Sample

A pair of samples was prepared by attaching wiring to a part of the upper surface of the prepared electrode pad A, and then laminating a pressuresensitive adhesive layer and a housing on the upper surface of the electrode pad A.

(3) Measurement of Impedance

The broken lines attached to the pair of samples were connected to a power source (impedance analyzer IM3570, manufactured by HIOKI Co., Ltd.), and the impedance was measured at a frequency of 10 Hz. When the impedance was 1 MΩ or less, the sample was suitable for measurement of electrocardiographs, and was evaluated as excellent.

3. Followability to Unevenness

As the followability to unevenness of the electrode pad, the size of the gap at the time of expansion and contraction was measured. The size of the gap during expansion and contraction was measured by cutting out a cross-section of the electrode pad, observing the cross-section using a microscope ("VHX", manufactured by KEYENCE Corporation), and measuring the size of the gap during expansion and contraction. In this observation, when the size of the gap at the time of expansion and contraction was 1.0 mm or less, the impedance could be adjusted to 1 MΩ or less, and the possibility of causing a problem in measurement of electrocardiograph was low. Therefore, the sample could be suitably used for measurement of electrocardiograph, and was evaluated as excellent.

4. Average Value of RR Intervals

Electrocardiographs were measured for 24 hours using the samples prepared as described above, and the average value of the RR intervals of the electrocardiograph obtained was calculated. In the case where there is no noise, an electrocardiograph waveform obtained by measuring the electrocardiograph is an electrocardiograph waveform constituted by a P wave, a QRS wave, and a T wave (see FIG. 5). It was evaluated that the higher the average value of the RR intervals, the more stably the electrocardiograph was measured, and when the average value of the RR intervals was 0.5 or more, the electrocardiograph was suitably measured, and was evaluated as excellent.

5. Presence or Absence of Peeling

Using the samples prepared as described above, electrocardiographs were observed for a measure of 24 hours to see whether the sample pads were peeled off from the skin of the subject, and the electrocardiographs were evaluated based on the following evaluation criteria. When the electrode pad was not peeled off from the skin of the subject and there was no problem, the result was determined to be excellent (denoted by A in Table 3). When the electrode pad was peeled off from the skin of the subject, the result was determined to be poor (denoted by B in Table 3).

Evaluation Criteria

A: Peeling of the electrode pad from the skin of the subject is not observed.
B: Peeling of the electrode pad from the skin of the subject is observed at least in a part.

[Examples 2 and 3, Comparative Examples 1 to 5]

The electrode pads were prepared in the same manner as in Example 1, except that the electrode sheets and second conductor layers were changed as indicated in Table 3.
Table 3 indicates the impedance, the size of the gap during expansion and contraction, the average RR intervals, and the presence or absence of peeling of the electrode pads in Examples and Comparative Examples.

[Table 3]

| | Electrode sheet | | Second conductive layer | | Electrode pad | | | | | |
|---|---|---|---|---|---|---|---|---|---|---|
| | Type | Materials | Type | Materials | Type | Polarization voltage [mV] | Impedance [MΩ] | Gap in expansion and contraction [mm] | RR interval (average value) | Present or absence of peeling |
| Example 1 | 1 | PEDOT/PSS | 1 | NaCl | A | 86 | 0.11 | 0.2 | 0.77 | A |
| Example 2 | 1 | PEDOT/PSS | 2 | KCl | B | 44 | 0.08 | 0.5 | 0.93 | A |
| Example 3 | 1 | PEDOT/PSS | 3 | CaCl | C | 11 | 0.04 | 0.8 | 0.86 | A |
| Example 4 | 6 | PEDOT/PSS | 1 | NaCl | A | 86 | 0.11 | 0.5 | 0.64 | A |
| Comparative Example 1 | 2 | PEDOT/PSS | 4 | NaCl | a | 131 | 1.30 | 1.2 | 0.45 | A |
| Comparative Example 2 | 3 | PEDOT/PSS | 5 | NaCl | b | 213 | 210 | 1.8 | 0.25 | A |
| Comparative Example 3 | 4 | PEDOT/PSS | 6 | NaCl | c | 248 | 2.80 | 0.1 | 0.34 | A |
| Comparative Example 4 | 5 | Cu | 7 | NaCl | d | 275 | 300 | × | 0.16 | A |
| Comparative Example 5 | 1 | PEDOT/PSS | - | - | e | 253 | 1.80 | 1.3 | 0.22 | A |
| Comparative Example 6 | 7 | PEDOT/PSS | 1 | NaCl | A | 86 | 0.11 | 1.2 | 0.22 | A |
| Comparative Example 7 | 8 | PEDOT/PSS | 1 | NaCl | A | 86 | 0.11 | 0.1 | 0.04 | B |

In Table 3, it was confirmed that all of the impedances, the followability to unevenness, the average value of the RR intervals, and the presence or absence of peeling of the electrode pads satisfied the conditions for use required for a living body sensor for measuring electrocardiographs in Examples 1 to 4. In contrast, it was confirmed that at least one or more of the impedances, the followability to unevenness, the average value of the RR intervals, and the presence or absence of peeling of the electrode pads did not satisfy the conditions for use required for a living body sensor for measuring electrocardiographs in Comparative Examples 1 to 7, and confirmed that there was a problem in practical use.

Therefore, the electrode pads of Examples 1 to 4 are different from the electrode pads of Comparative Examples 1 to 7 in that the elongation at break of the electrode sheet is 50% to 600% and the second conductive layer contains 0.5% by mass to 12.5% by mass of a metal chloride. As a result, the low polarization voltage, low impedance, and stable electrocardiograph waveform can be acquired by suppressing noise generated in the electrocardiograph, and also, the followability to unevenness of skin is excellent, and the state of being stably attached to the skin can be maintained. Thus, it can be said that the electrode pad of the present embodiment can be used effectively for stable measurement of a subject's electrocardiograph even if the electrode pad is attached to the subject's skin for a long period of time (e.g., 24 hours) using the electrode pad as a living body sensor.

The embodiment has been described above, but the embodiment is presented as an example, and the present invention is not limited to the embodiment.

The above-described embodiments can be implemented in various other forms, and various combinations, omissions, substitutions, changes, and the like can be made without departing from the spirit of the invention. These embodiments and modifications thereof are included in the scope and spirit of the invention, and are included in the invention described in the claims and the scope of equivalents thereof.

Aspects of the embodiments of the present invention are as follows, for example.

<1> An electrode pad includes: a first conductive layer; and a second conductive layer provided on one surface of the first conductive layer, wherein the first conductive layer has an elongation at break of 50% to 600%, and wherein the second conductive layer contains 0.1% by mass to 40% by mass of a metal chloride.

<2> The electrode pad according to <1>, wherein an impedance with skin is $1M\Omega$ or less.

<3> The electrode pad according to <1> or <2>, wherein a polarization voltage is in a range from 0.1 mV to 100 mV.

<4> The electrode pad according to any one of <1> to <3>, wherein the second conductive layer contains a pseudoplastic fluid or a Bingham fluid.

<5> The electrode pad according to <4>, wherein the second conductive layer has a viscosity of 1 mPas to 100,000 mPas.

<6> The electrode pad according to any one of <1> to <5>, wherein a metal of the metal chloride contains one or more components selected from the group consisting of Cu, Na, Sn, Al, Si, K, Ag, and Ca.

<7> The electrode pad according to any one of <1> to <6>, wherein the first conductive layer is a bioelectrode.

<8> A living body sensor used by being attached to a surface of a living body, includes: a base adhesive layer provided on a surface on the living body side of a foam substrate having a porous structure; and an electrode pad provided on a surface on the living body side of the base adhesive layer, wherein the electrode pad is the electrode pad of any one of <1> to <7>.

<9> The living body sensor according to <8>, wherein the second conductive layer of the electrode pad is provided up to the base adhesive layer around the electrode pad.

<10> The living body sensor according to <8> or <9>, further includes: a base on which a sensor unit is provided, the sensor unit being connected to the electrode pad and configured to acquire biometric information; and an attachment adhesive layer provided on a surface of the base close to the living body, wherein the base adhesive layer, the electrode pad, and the attachment adhesive layer form an attachment surface to the living body.

<11> The living body sensor according to any one of <8> to <10>, wherein the first conductive layer has a through hole, and wherein the base adhesive layer is exposed from the through hole.

This application claims priority under Japanese Patent Application No. 2021-159960, filed on September 29, 2021, and the entire contents of which are hereby incorporated by reference.

DESCRIPTION OF THE REFERENCE NUMERALS

1, 23: electrode pad
2a: skin
3: adhesive layer
11, 231: first conductive layer (electrode)

111 231A: through hole
12, 232: second conductive layer
20: living body sensor
21: housing (cover member)
22: foam sheet
221: foam base
222: base adhesive layer
223: housing adhesive layer
24: supporting adhesive sheet
241: base
242: attachment adhesive layer
243: sensor adhesive layer
25: sensor unit

## Claims

1. An electrode pad comprising:

   a first conductive layer; and
   a second conductive layer provided on one surface of the first conductive layer,
   wherein the first conductive layer has an elongation at break of 50% to 600%, and
   wherein the second conductive layer contains 0.1% by mass to 40% by mass of a metal chloride.

2. The electrode pad according to claim 1, wherein an impedance with skin is 1 MΩ or less.

3. The electrode pad according to claim 1, wherein a polarization voltage is in a range from 0.1 mV to 100 mV.

4. The electrode pad according to claim 1, wherein the second conductive layer contains a pseudoplastic fluid or a Bingham fluid.

5. The electrode pad according to claim 4, wherein the second conductive layer has a viscosity of 1 mPas to 100,000 mPas.

6. The electrode pad according to claim 1, wherein a metal of the metal chloride contains one or more components selected from the group consisting of Cu, Na, Sn, Al, Si, K, Ag, and Ca.

7. The electrode pad according to claim 1, wherein the first conductive layer is a bioelectrode.

8. A living body sensor used by being attached to a surface of a living body, comprising:

   a base adhesive layer provided on a surface on the living body side of a foam substrate having a porous structure; and
   an electrode pad provided on a surface on the living body side of the base adhesive layer,
   wherein the electrode pad is the electrode pad of claim 1.

9. The living body sensor according to claim 8, wherein the second conductive layer of the electrode pad is provided up to the base adhesive layer around the electrode pad.

10. The living body sensor according to claim 8, further comprising:

    a base on which a sensor unit is provided, the sensor unit being connected to the electrode pad and configured to acquire biometric information; and
    an attachment adhesive layer provided on a surface of the base close to the living body,
    wherein the base adhesive layer, the electrode pad, and the attachment adhesive layer form an attachment surface to the living body.

11. The living body sensor according to claim 8,

wherein the first conductive layer has a through hole, and
wherein the base adhesive layer is exposed from the through hole.

# FIG.1

# FIG.2

# FIG.3

# FIG.4

# FIG.5

## ELECTROCARDIOGRAPHIC WAVEFORM WITHOUT NOISE

# FIG.6

# FIG.7

# FIG.8

EP 4 410 206 A1

# FIG.9

<table>
<tr><td colspan="2"><div align="center"><strong>INTERNATIONAL SEARCH REPORT</strong></div></td><td>International application No.<br><br><strong>PCT/JP2022/035748</strong></td></tr>
</table>

**A.      CLASSIFICATION OF SUBJECT MATTER**

*A61B 5/257*(2021.01)i; *A61B 5/263*(2021.01)i; *H01B 1/20*(2006.01)i
FI:    A61B5/257; A61B5/263; H01B1/20 B

According to International Patent Classification (IPC) or to both national classification and IPC

**B.      FIELDS SEARCHED**

Minimum documentation searched (classification system followed by classification symbols)

A61B5/05-5/0538, 5/24-5/398

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.      DOCUMENTS CONSIDERED TO BE RELEVANT**

| Category* | Citation of document, with indication, where appropriate, of the relevant passages | Relevant to claim No. |
|---|---|---|
| Y | JP 2019-51236 A (OSAKA UNIV.) 04 April 2019 (2019-04-04)<br>paragraphs [0026], [0029], [0060], [0067], [0097], [0100], [0102] | 1-11 |
| Y | JP 2020-55918 A (TAIYO INK MFG. LTD.) 09 April 2020 (2020-04-09)<br>paragraph [0052] | 1-11 |
| Y | WO 2019/239996 A1 (NOK CORP.) 19 December 2019 (2019-12-19)<br>paragraphs [0056]-[0057], fig. 15 | 2-3 |
| Y | JP 2010-65222 A (TYCO HEALTHCARE GROUP LP) 25 March 2010 (2010-03-25)<br>paragraphs [0016], [0023], [0052], [0073] | 4-5 |
| Y | WO 2020/184346 A1 (NITTO DENKO CORP.) 17 September 2020 (2020-09-17)<br>paragraphs [0026], [0032], [0096]-[0097], fig. 1-2 | 8-11 |
| A | JP 2003-339654 A (TOYO INK MFG. CO., LTD.) 02 December 2003 (2003-12-02)<br>paragraphs [0016]-[0017], [0031], [0037], [0046], [0050] | 1-11 |

☐ Further documents are listed in the continuation of Box C.          ☑ See patent family annex.

| | |
|---|---|
| \*      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **11 October 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<table>
<tr><td colspan="3" align="center"><b>INTERNATIONAL SEARCH REPORT</b><br><b>Information on patent family members</b></td><td colspan="2">International application No.<br><br><b>PCT/JP2022/035748</b></td></tr>
</table>

| Patent document<br>cited in search report | | | Publication date<br>(day/month/year) | Patent family member(s) | Publication date<br>(day/month/year) |
|---|---|---|---|---|---|
| JP | 2019-51236 | A | 04 April 2019 | WO 2019/058739 A1 | |
| JP | 2020-55918 | A | 09 April 2020 | (Family: none) | |
| WO | 2019/239996 | A1 | 19 December 2019 | US 2020/0251258 A1<br>paragraphs [0091]-[0093], fig. 15<br>EP 3808261 A1<br>CN 111225607 A | |
| JP | 2010-65222 | A | 25 March 2010 | US 2010/0059722 A1<br>paragraphs [0014], [0021], [0050], [0064]<br>EP 2163193 A1 | |
| WO | 2020/184346 | A1 | 17 September 2020 | EP 3936041 A1<br>paragraphs [0028], [0032], [0096]-[0097], fig. 1-2<br>CN 113597284 A | |
| JP | 2003-339654 | A | 02 December 2003 | (Family: none) | |

Form PCT/ISA/210 (patent family annex) (January 2015)

**EP 4 410 206 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020156692 A
- JP 2021159960 A